(19) Europäisches Patentamt
European Patent Office
Office européen des brevets

(11) Publication number: **0 276 558 B1**

(12) **EUROPEAN PATENT SPECIFICATION**

(45) Date of publication of patent specification: **08.07.92**

(51) Int. Cl.[5]: **C07C 43/29**, C07C 43/295, C07C 43/23, C07C 49/84, C07C 255/00, C07D 303/22, C07C 39/27, C07C 43/225, A01N 31/14, A01N 37/34

(21) Application number: **87311027.4**

(22) Date of filing: **15.12.87**

The file contains technical information submitted after the application was filed and not included in this specification

# (54) Insecticidal ethers.

(30) Priority: **08.01.87 GB 8700393**
**08.01.87 GB 8700394**

(43) Date of publication of application:
**03.08.88 Bulletin 88/31**

(45) Publication of the grant of the patent:
**08.07.92 Bulletin 92/28**

(84) Designated Contracting States:
**AT BE CH DE ES FR GR IT LI LU NL SE**

(56) References cited:
**EP-A- 0 179 018**
**EP-A- 0 211 561**
**EP-A- 0 233 834**
**DE-A- 2 616 479**
**US-A- 4 518 604**

(73) Proprietor: **IMPERIAL CHEMICAL INDUSTRIES PLC**
**Imperial Chemical House, Millbank**
**London SW1P 3JF(GB)**

(72) Inventor: **Bushell, Michael John**
**21 Suffolk Close Woosehill**
**Wokingham Berkshire(GB)**
Inventor: **Carr, Robin Arthur Ellis**
**4 Sandy Lane Little Sandhurst**
**Camberley Surrey(GB)**

(74) Representative: **Bishop, Nigel Douglas et al**
**ICI Group Patents Services Dept. PO Box 6**
**Shire Park Bessemer Road**
**Welwyn Garden City Herts, AL7 1HD(GB)**

EP 0 276 558 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

TETRAHEDRON, vol. 33, 1977, pages 991-994, Pergamon Press, GB; E. G. SUNDHOLM, "Isomerisation of benzophenones and direct observation of intermediates in the aromatic acylation of phloroglucinol derivatives with trifluoroacetic anhydride"

JOURNAL OF ORGANOMETALLIC CHEMISTRY, vol. 292, nos. 1/2, 3rd September 1985, pages 145-149, Elsevier Sequoia S.A.; O. A. VYAZANKINA et al, "Cyanide-ion-catalyzed reaction of pentafluorophenyltrimethylsilane with substituted acetophenones"

## Description

This invention relates to novel fluorinated ethers, useful as insecticides and acaricides, to processes and intermediates for their preparation, to insecticidal and acaricidal compositions comprising them and to methods of combating and controlling insect and acarine pests therewith.

Insecticidal activity is disclosed for 3,3-difluorinated 2-arylpropyl ethers in European Patent Application No. 0179018-A. In the continuing search for novel compounds exhibiting insecticidal activity, the present applicants have demonstrated insecticidal properties for certain 3,3,3-trifluorinated 2-phenylpropyl ethers.

In a first aspect the invention provides compounds of formula (I) :

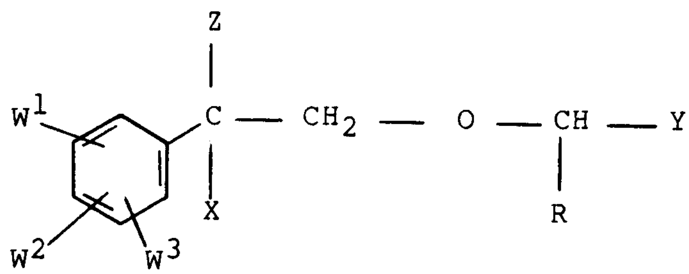

(I)

wherein X is selected from hydrogen, halo, hydroxy, alkoxy of up to four carbon atoms and acyloxy of up to four carbon atoms, Z represents a fluoroalkyl group of one or two carbon atoms, Y represents a substituted aryl group where each substituent is selected from halo, alkyl of up to six carbon atoms, aryl, aralkyl of up to four carbon atoms in the alkyl moiety, aryloxy and arylamino, and either

(i) R represents hydrogen, and $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms, and haloalkoxy of up to six carbon atoms, or

(ii) R is selected from methyl, trifluoromethyl, cyano and ethynyl, and $W^1$, $W^2$ and $W^3$ are independently selected from hydrogen, halo, alkyl of up to six carbon atoms, alkoxy of up to six carbon atoms, alkoxyalkyl of up to a total of six carbon atoms, haloalkyl of up to six carbon atoms, and haloalkoxy of up to six carbon atoms.

Preferred compounds according to the invention are those according to formula I as described hereinbefore wherein X is selected from hydrogen, halo and hydroxy, Z represents the trifluoromethyl group, Y represents an aryl group selected from phenyl, pyridyl and furyl, substituted with one or more substituents selected from fluoro, methyl, phenyl, benzyl, phenoxy, chlorophenoxy, fluorophenoxy, bromophenoxy and fluoroanilino, and either

(i) R represents hydrogen, and $W^1$, $W^2$ and $W^3$ are independently selected from fluoro, chloro, bromo, alkyl of up to four carbon atoms, alkoxy of up to four carbon atoms, alkoxyalkyl of up to a total of four carbon atoms, haloalkyl of up to two carbon atoms and haloalkoxy of up to two carbon atoms, or

(ii) R is selected from methyl, trifluoromethyl, cyano and ethynyl, and $W^1$, $W^2$ and $W^3$ are independently selected from hydrogen, fluoro, chloro, bromo, alkyl of up to four carbon atoms, alkoxy of up to four carbon atoms, alkoxyalkyl of up to a total of four carbon atoms, haloalkyl of up to two carbon atoms and haloalkoxy of up to two carbon atoms.

Particularly preferred compounds according to the invention are those according to formula (I) as described hereinbefore wherein X is selected from hydrogen, fluoro and chloro, Z represents trifluoromethyl, Y represents a phenoxyphenyl group in which each phenyl group may be unsubstituted or substituted with halogen, and R, $W^1$, $W^2$ and $W^3$ have any of the meanings given hereinbefore, with the proviso that one of $W^1$, $W^2$ and $W^3$ represents a substituent in the 4-position selected from chloro, fluoro, methoxy, ethoxy, trifluoromethyl, trifluoromethoxy and difluoromethoxy.

Particular examples of compounds according to the invention include those set out in Table I below, wherein the compounds correspond to the formula :

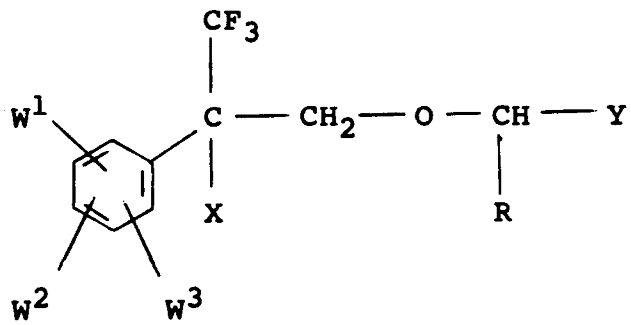

In Table I, Y is defined as $R^1$ to $R^{14}$, wherein $R^1$ to $R^{14}$ represent the following groups :

$R^1$ : 3-phenoxyphenyl
$R^2$ : 3-(4-chlorophenoxy)phenyl
$R^3$ : 4-fluoro-3-phenoxyphenyl
$R^4$ : 3-(4-bromophenoxy)phenyl
$R^5$ : 4-fluoro-3-(4-bromophenoxy)phenyl
$R^6$ : 4-fluoro-3-(4-chlorophenoxy)phenyl
$R^7$ : 3-(2,4-difluorophenoxy)phenyl
$R^8$ : 3-benzylphenyl
$R^9$ : 3-benzyl-4-fluorophenyl
$R^{10}$ : 3-(4-fluorophenylamino)phenyl
$R^{11}$ : 6-phenoxypyrid-2-yl
$R^{12}$ : 2-methyl-3-phenylphenyl
$R^{13}$ : 4-methyl-2,3,5,6-tetrafluorophenyl
$R^{14}$ : 5-benzylfuran-3-yl

TABLE I

| Compound No | $W^1$ | $W^2$ | $W^3$ | X | R | Y |
|---|---|---|---|---|---|---|
| 1 | $4-OC_2H_5$ | 3-F | 5-F | H | H | $R^1$ |
| 2 | $4-OC_2H_5$ | 3-F | 5-F | H | H | $R^6$ |
| 3 | $4-OC_2H_5$ | 3-F | 5-F | H | H | $R^2$ |
| 4 | $4-OC_2H_5$ | 3-F | 5-F | H | H | $R^3$ |
| 5 | $4-OC_2H_5$ | 3-F | 5-F | H | H | $R^8$ |
| 6 | $4-OC_2H_5$ | 3-F | 5-F | H | H | $R^9$ |
| 7 | $4-OC_2H_5$ | 3-F | 5-F | H | H | $R^{11}$ |
| 8 | $4-OC_2H_5$ | 3-F | 5-F | H | H | $R^4$ |
| 9 | $4-OC_2H_5$ | 3-F | 5-F | H | H | $R^5$ |
| 10 | $4-OC_2H_5$ | 3-F | 5-F | H | H | $R^7$ |
| 11 | $4-OC_2H_5$ | 3-F | 5-F | H | H | $R^{10}$ |
| 12 | $4-OC_2H_5$ | 3-F | 5-F | H | H | $R^{12}$ |
| 13 | $4-OC_2H_5$ | 3-F | 5-F | H | H | $R^{13}$ |
| 14 | $4-OC_2H_5$ | 3-F | 5-F | OH | H | $R^1$ |
| 15 | $4-OC_2H_5$ | 3-F | 5-F | OH | H | $R^6$ |
| 16 | $4-OC_2H_5$ | 3-F | 5-F | OH | H | $R^2$ |
| 17 | $4-OC_2H_5$ | 3-F | 5-F | OH | H | $R^3$ |
| 18 | $4-OC_2H_5$ | 3-F | 5-F | OH | H | $R^8$ |

TABLE I CONTINUED

| Compound No | $W^1$ | $W^2$ | $W^3$ | X | R | Y |
|---|---|---|---|---|---|---|
| 19 | 4-$OC_2H_5$ | 3-F | 5-F | OH | H | $R^9$ |
| 20 | 4-$OC_2H_5$ | 3-F | 5-F | OH | H | $R^{11}$ |
| 21 | 4-$OC_2H_5$ | 3-F | 5-F | Cl | H | $R^1$ |
| 22 | 4-$OC_2H_5$ | 3-F | 5-F | Cl | H | $R^6$ |
| 23 | 4-$OC_2H_5$ | 3-F | 5-F | Cl | H | $R^2$ |
| 24 | 4-$OC_2H_5$ | 3-F | 5-F | Cl | H | $R^3$ |
| 25 | 4-$OC_2H_5$ | 3-F | 5-F | Cl | H | $R^8$ |
| 26 | 4-$OC_2H_5$ | 3-F | 5-F | Cl | H | $R^9$ |
| 27 | 4-$OC_2H_5$ | 3-F | 5-F | Cl | H | $R^{11}$ |
| 28 | 4-$OC_2H_5$ | 3-F | 5-F | F | H | $R^1$ |
| 29 | 4-$OC_2H_5$ | 3-F | 5-F | F | H | $R^6$ |
| 30 | 4-$OC_2H_5$ | 3-F | 5-F | F | H | $R^2$ |
| 31 | 4-$OC_2H_5$ | 3-F | 5-F | F | H | $R^3$ |
| 32 | 4-$OC_2H_5$ | 3-F | 5-F | F | H | $R^8$ |
| 33 | 4-$OC_2H_5$ | 3-F | 5-F | F | H | $R^9$ |
| 34 | 4-$OC_2H_5$ | 3-F | 5-F | F | H | $R^{11}$ |
| 35 | 4-$OCF_3$ | 3-F | 5-F | H | H | $R^1$ |
| 36 | 4-$OCF_3$ | 3-F | 5-F | H | H | $R^6$ |
| 37 | 4-$OCF_3$ | 3-F | 5-F | H | H | $R^2$ |
| 38 | 4-$OCF_3$ | 3-F | 5-F | H | H | $R^3$ |
| 39 | 4-$OCF_3$ | 3-F | 5-F | H | H | $R^8$ |
| 40 | 4-$OCF_3$ | 3-F | 5-F | H | H | $R^9$ |
| 41 | 4-$OCF_3$ | 3-F | 5-F | H | H | $R^{11}$ |
| 42 | 4-$OCF_3$ | 3-F | 5-F | H | H | $R^4$ |
| 43 | 4-$OCF_3$ | 3-F | 5-F | H | H | $R^5$ |

TABLE I CONTINUED

| Compound No | $W^1$ | $W^2$ | $W^3$ | X | R | Y |
|---|---|---|---|---|---|---|
| 44 | 4-$OCF_3$ | 3-F | 5-F | H | H | $R^7$ |
| 45 | 4-$OCF_3$ | 3-F | 5-F | H | H | $R^{10}$ |
| 46 | 4-$OCF_3$ | 3-F | 5-F | H | H | $R^{12}$ |
| 47 | 4-$OCF_3$ | 3-F | 5-F | H | H | $R^{13}$ |
| 48 | 4-$OCF_3$ | 3-F | 5-F | OH | H | $R^1$ |
| 49 | 4-$OCF_3$ | 3-F | 5-F | OH | H | $R^6$ |
| 50 | 4-$OCF_3$ | 3-F | 5-F | OH | H | $R^2$ |
| 51 | 4-$OCF_3$ | 3-F | 5-F | OH | H | $R^3$ |
| 52 | 4-$OCF_3$ | 3-F | 5-F | OH | H | $R^8$ |
| 53 | 4-$OCF_3$ | 3-F | 5-F | OH | H | $R^9$ |
| 54 | 4-$OCF_3$ | 3-F | 5-F | OH | H | $R^{11}$ |
| 55 | 4-$OCF_3$ | 3-F | 5-F | F | H | $R^1$ |
| 56 | 4-$OCF_3$ | 3-F | 5-F | F | H | $R^6$ |
| 57 | 4-$OCF_3$ | 3-F | 5-F | F | H | $R^2$ |
| 58 | 4-$OCF_3$ | 3-F | 5-F | F | H | $R^3$ |
| 59 | 4-$OCF_3$ | 3-F | 5-F | F | H | $R^8$ |
| 60 | 4-$OCF_3$ | 3-F | 5-F | F | H | $R^9$ |
| 61 | 4-$OCF_3$ | 3-F | 5-F | F | H | $R^{11}$ |
| 62 | 4-$OCF_3$ | 3-F | 5-F | Cl | H | $R^1$ |
| 63 | 4-$OCF_3$ | 3-F | 5-F | Cl | H | $R^6$ |
| 64 | 4-$OCF_3$ | 3-F | 5-F | Cl | H | $R^2$ |
| 65 | 4-$OCF_3$ | 3-F | 5-F | Cl | H | $R^3$ |
| 66 | 4-$OCF_3$ | 3-F | 5-F | Cl | H | $R^8$ |
| 67 | 4-$OCF_3$ | 3-F | 5-F | Cl | H | $R^9$ |
| 68 | 4-$OCF_3$ | 3-F | 5-F | Cl | H | $R^{11}$ |

TABLE I CONTINUED

| Compound No | $W^1$ | $W^2$ | $W^3$ | X | R | Y |
|---|---|---|---|---|---|---|
| 69 | 4-$OCHF_2$ | 3-F | 5-F | H | H | $R^1$ |
| 70 | 4-$OCHF_2$ | 3-F | 5-F | H | H | $R^6$ |
| 71 | 4-$OCHF_2$ | 3-F | 5-F | H | H | $R^2$ |
| 72 | 4-$OCHF_2$ | 3-F | 5-F | H | H | $R^3$ |
| 73 | 4-$OCHF_2$ | 3-F | 5-F | H | H | $R^8$ |
| 74 | 4-$OCHF_2$ | 3-F | 5-F | H | H | $R^9$ |
| 75 | 4-$OCHF_2$ | 3-F | 5-F | H | H | $R^{11}$ |
| 76 | 4-$OCHF_2$ | 3-F | 5-F | F | H | $R^1$ |
| 77 | 4-$OCHF_2$ | 3-F | 5-F | F | H | $R^6$ |
| 78 | 4-$OCHF_2$ | 3-F | 5-F | F | H | $R^2$ |
| 79 | 4-$OCHF_2$ | 3-F | 5-F | F | H | $R^3$ |
| 80 | 4-$OCHF_2$ | 3-F | 5-F | F | H | $R^8$ |
| 81 | 4-$OCHF_2$ | 3-F | 5-F | F | H | $R^9$ |
| 82 | 4-$OCHF_2$ | 3-F | 5-F | F | H | $R^{11}$ |
| 83 | 4-$OCHF_2$ | 3-F | 5-F | Cl | H | $R^1$ |
| 84 | 4-$OCHF_2$ | 3-F | 5-F | Cl | H | $R^6$ |
| 85 | 4-$OCHF_2$ | 3-F | 5-F | Cl | H | $R^2$ |
| 86 | 4-$OCHF_2$ | 3-F | 5-F | Cl | H | $R^3$ |
| 87 | 4-$OCHF_2$ | 3-F | 5-F | Cl | H | $R^8$ |
| 88 | 4-$OCHF_2$ | 3-F | 5-F | Cl | H | $R^9$ |
| 89 | 4-$OCHF_2$ | 3-F | 5-F | Cl | H | $R^{11}$ |
| 90 | 4-$OC_2H_5$ | H | H | H | CN | $R^1$ |
| 91 | 4-$OC_2H_5$ | H | H | Cl | CN | $R^1$ |
| 92 | 4-$OC_2H_5$ | H | H | F | CN | $R^1$ |
| 93 | 4-$OC_2H_5$ | H | H | OH | CN | $R^1$ |
| 94 | 4-$OC_2H_5$ | H | H | H | $CH_3$ | $R^1$ |

TABLE I CONTINUED

| Compound No | $W^1$ | $W^2$ | $W^3$ | X | R | Y |
|---|---|---|---|---|---|---|
| 95 | $4-OC_2H_5$ | H | H | Cl | $CH_3$ | $R^1$ |
| 96 | $4-OC_2H_5$ | H | H | F | $CH_3$ | $R^1$ |
| 97 | $4-OC_2H_5$ | H | H | OH | $CH_3$ | $R^1$ |
| 98 | $4-OC_2H_5$ | H | H | H | $-C\equiv CH$ | $R^1$ |
| 99 | $4-OC_2H_5$ | H | H | Cl | $-C\equiv CH$ | $R^1$ |
| 100 | $4-OC_2H_5$ | H | H | F | $-C\equiv CH$ | $R^1$ |
| 101 | $4-OC_2H_5$ | H | H | OH | $-C\equiv CH$ | $R^1$ |
| 102 | $4-OC_2H_5$ | H | H | H | $CF_3$ | $R^1$ |
| 103 | $4-OC_2H_5$ | H | H | Cl | $CF_3$ | $R^1$ |
| 104 | $4-OC_2H_5$ | H | H | F | $CF_3$ | $R^1$ |
| 105 | $4-OC_2H_5$ | H | H | OH | $CF_3$ | $R^1$ |
| 106 | $4-OC_2H_5$ | H | H | H | CN | $R^6$ |
| 107 | $4-OC_2H_5$ | H | H | Cl | CN | $R^6$ |
| 108 | $4-OC_2H_5$ | H | H | F | CN | $R^6$ |
| 109 | $4-OC_2H_5$ | H | H | OH | CN | $R^6$ |
| 110 | $4-OC_2H_5$ | H | H | H | $CH_3$ | $R^6$ |
| 111 | $4-OC_2H_5$ | H | H | Cl | $CH_3$ | $R^6$ |
| 112 | $4-OC_2H_5$ | H | H | F | $CH_3$ | $R^6$ |
| 113 | $4-OC_2H_5$ | H | H | OH | $CH_3$ | $R^6$ |
| 114 | $4-OC_2H_5$ | H | H | H | $CF_3$ | $R^6$ |
| 115 | $4-OC_2H_5$ | H | H | Cl | $CF_3$ | $R^6$ |
| 116 | $4-OC_2H_5$ | H | H | F | $CF_3$ | $R^6$ |
| 117 | $4-OC_2H_5$ | H | H | OH | $CF_3$ | $R^6$ |

TABLE I CONTINUED

| Compound No | $W^1$ | $W^2$ | $W^3$ | X | R | Y |
|---|---|---|---|---|---|---|
| 118 | 4-$OC_2H_5$ | H | H | H | -C≡CH | $R^6$ |
| 119 | 4-$OC_2H_5$ | H | H | Cl | -C≡CH | $R^6$ |
| 120 | 4-$OC_2H_5$ | H | H | F | -C≡CH | $R^6$ |
| 121 | 4-$OC_2H_5$ | H | H | OH | -C≡CH | $R^6$ |
| 122 | 4-$OC_2H_5$ | H | H | H | CN | $R^3$ |
| 123 | 4-$OC_2H_5$ | H | H | Cl | CN | $R^3$ |
| 124 | 4-$OC_2H_5$ | H | H | F | CN | $R^3$ |
| 125 | 4-$OC_2H_5$ | H | H | OH | CN | $R^3$ |
| 126 | 4-$OC_2H_5$ | H | H | H | $CH_3$ | $R^3$ |
| 127 | 4-$OC_2H_5$ | H | H | Cl | $CH_3$ | $R^3$ |
| 128 | 4-$OC_2H_5$ | H | H | F | $CH_3$ | $R^3$ |
| 129 | 4-$OC_2H_5$ | H | H | OH | $CH_3$ | $R^3$ |
| 130 | 4-$OC_2H_5$ | H | H | H | $CF_3$ | $R^3$ |
| 131 | 4-$OC_2H_5$ | H | H | Cl | $CF_3$ | $R^3$ |
| 132 | 4-$OC_2H_5$ | H | H | F | $CF_3$ | $R^3$ |
| 133 | 4-$OC_2H_5$ | H | H | OH | $CF_3$ | $R^3$ |
| 134 | 4-$OC_2H_5$ | H | H | H | -C≡CH | $R^3$ |
| 135 | 4-$OC_2H_5$ | H | H | Cl | -C≡CH | $R^3$ |
| 136 | 4-$OC_2H_5$ | H | H | F | -C≡CH | $R^3$ |
| 137 | 4-$OC_2H_5$ | H | H | OH | -C≡CH | $R^3$ |
| 138 | 4-$OC_2H_5$ | H | H | H | CN | $R^2$ |
| 139 | 4-$OC_2H_5$ | H | H | Cl | CN | $R^2$ |
| 140 | 4-$OC_2H_5$ | H | H | F | CN | $R^2$ |
| 141 | 4-$OC_2H_5$ | H | H | OH | CN | $R^2$ |
| 142 | 4-$OC_2H_5$ | H | H | H | $CH_3$ | $R^2$ |
| 143 | 4-$OC_2H_5$ | H | H | Cl | $CH_3$ | $R^2$ |
| 144 | 4-$OC_2H_5$ | H | H | F | $CH_3$ | $R^2$ |

TABLE I CONTINUED

| Compound No | $W^1$ | $W^2$ | $W^3$ | X | R | Y |
|---|---|---|---|---|---|---|
| 145 | $4-OC_2H_5$ | H | H | OH | $CH_3$ | $R^2$ |
| 146 | $4-OC_2H_5$ | H | H | H | $CF_3$ | $R^2$ |
| 147 | $4-OC_2H_5$ | H | H | Cl | $CF_3$ | $R^2$ |
| 148 | $4-OC_2H_5$ | H | H | F | $CF_3$ | $R^2$ |
| 149 | $4-OC_2H_5$ | H | H | OH | $CF_3$ | $R^2$ |
| 150 | $4-OC_2H_5$ | H | H | H | $-C\equiv CH$ | $R^2$ |
| 151 | $4-OC_2H_5$ | H | H | Cl | $-C\equiv CH$ | $R^2$ |
| 152 | $4-OC_2H_5$ | H | H | F | $-C\equiv CH$ | $R^2$ |
| 153 | $4-OC_2H_5$ | H | H | OH | $-C\equiv CH$ | $R^2$ |
| 154 | $4-OCF_3$ | H | H | H | CN | $R^2$ |
| 155 | $4-OCF_3$ | H | H | Cl | CN | $R^2$ |
| 156 | $4-OCF_3$ | H | H | F | CN | $R^2$ |
| 157 | $4-OCF_3$ | H | H | OH | CN | $R^2$ |
| 158 | $4-OCF_3$ | H | H | H | $CF_3$ | $R^2$ |
| 159 | $4-OCF_3$ | H | H | Cl | $CF_3$ | $R^2$ |
| 160 | $4-OCF_3$ | H | H | F | $CF_3$ | $R^2$ |
| 161 | $4-OCF_3$ | H | H | OH | $CF_3$ | $R^2$ |
| 162 | $4-OCF_3$ | H | H | H | $CH_3$ | $R^2$ |
| 163 | $4-OCF_3$ | H | H | Cl | $CH_3$ | $R^2$ |
| 164 | $4-OCF_3$ | H | H | F | $CH_3$ | $R^2$ |
| 165 | $4-OCF_3$ | H | H | OH | $CH_3$ | $R^2$ |
| 166 | $4-OCF_3$ | H | H | H | $-C\equiv CH$ | $R^2$ |
| 167 | $4-OCF_3$ | H | H | Cl | $-C\equiv CH$ | $R^2$ |
| 168 | $4-OCF_3$ | H | H | F | $-C\equiv CH$ | $R^2$ |
| 169 | $4-OCF_3$ | H | H | OH | $-C\equiv CH$ | $R^2$ |
| 170 | $4-OCF_3$ | H | H | H | CN | $R^6$ |
| 171 | $4-OCF_3$ | H | H | Cl | CN | $R^6$ |
| 172 | $4-OCF_3$ | H | H | F | CN | $R^6$ |

TABLE I CONTINUED

| Compound No | $W^1$ | $W^2$ | $W^3$ | X | R | Y |
|---|---|---|---|---|---|---|
| 173 | 4-$OCF_3$ | H | H | OH | CN | $R^6$ |
| 174 | 4-$OCF_3$ | H | H | H | $CH_3$ | $R^6$ |
| 175 | 4-$OCF_3$ | H | H | Cl | $CH_3$ | $R^6$ |
| 176 | 4-$OCF_3$ | H | H | F | $CH_3$ | $R^6$ |
| 177 | 4-$OCF_3$ | H | H | OH | $CH_3$ | $R^6$ |
| 178 | 4-$OCF_3$ | H | H | H | $CF_3$ | $R^6$ |
| 179 | 4-$OCF_3$ | H | H | Cl | $CF_3$ | $R^6$ |
| 180 | 4-$OCF_3$ | H | H | F | $CF_3$ | $R^6$ |
| 181 | 4-$OCF_3$ | H | H | OH | $CF_3$ | $R^6$ |
| 182 | 4-$OCF_3$ | H | H | H | -C≡CH | $R^6$ |
| 183 | 4-$OCF_3$ | H | H | Cl | -C≡CH | $R^6$ |
| 184 | 4-$OCF_3$ | H | H | F | -C≡CH | $R^6$ |
| 185 | 4-$OCF_3$ | H | H | OH | -C≡CH | $R^6$ |
| 186 | 4-$OCF_3$ | H | H | H | CN | $R^1$ |
| 187 | 4-$OCF_3$ | H | H | Cl | CN | $R^1$ |
| 188 | 4-$OCF_3$ | H | H | F | CN | $R^1$ |
| 189 | 4-$OCF_3$ | H | H | H | $CH_3$ | $R^1$ |
| 190 | 4-$OCF_3$ | H | H | Cl | $CH_3$ | $R^1$ |
| 191 | 4-$OCF_3$ | H | H | F | $CH_3$ | $R^1$ |
| 192 | 4-$OCF_3$ | H | H | H | CN | $R^3$ |
| 193 | 4-$OCF_3$ | H | H | Cl | CN | $R^3$ |
| 194 | 4-$OCF_3$ | H | H | F | CN | $R^3$ |
| 195 | 4-$OCF_3$ | H | H | H | $CH_3$ | $R^3$ |
| 196 | 4-$OCF_3$ | H | H | Cl | $CH_3$ | $R^3$ |
| 197 | 4-$OCF_3$ | H | H | F | $CH_3$ | $R^3$ |
| 198 | 4-$OC_2H_5$ | 3-F | H | H | CN | $R^1$ |
| 199 | 4-$OC_2H_5$ | 3-F | H | Cl | CN | $R^1$ |
| 200 | 4-$OC_2H_5$ | 3-F | H | F | CN | $R^1$ |

TABLE I CONTINUED

| Compound No | $W^1$ | $W^2$ | $W^3$ | X | R | Y |
|---|---|---|---|---|---|---|
| 201 | $4\text{-}OC_2H_5$ | 3-F | H | H | $CH_3$ | $R^1$ |
| 202 | $4\text{-}OC_2H_5$ | 3-F | H | Cl | $CH_3$ | $R^1$ |
| 203 | $4\text{-}OC_2H_5$ | 3-F | H | F | $CH_3$ | $R^1$ |
| 204 | $4\text{-}OC_2H_5$ | 3-F | H | H | CN | $R^2$ |
| 205 | $4\text{-}OC_2H_5$ | 3-F | H | Cl | CN | $R^2$ |
| 206 | $4\text{-}OC_2H_5$ | 3-F | H | F | CN | $R^2$ |
| 207 | $4\text{-}OC_2H_5$ | 3-F | H | H | $CH_3$ | $R^2$ |
| 208 | $4\text{-}OC_2H_5$ | 3-F | H | Cl | $CH_3$ | $R^2$ |
| 209 | $4\text{-}OC_2H_5$ | 3-F | H | F | $CH_3$ | $R^2$ |
| 210 | $4\text{-}OC_2H_5$ | 3-F | H | H | CN | $R^6$ |
| 211 | $4\text{-}OC_2H_5$ | 3-F | H | Cl | CN | $R^6$ |
| 212 | $4\text{-}OC_2H_5$ | 3-F | H | F | CN | $R^6$ |
| 213 | $4\text{-}OC_2H_5$ | 3-F | H | H | $CH_3$ | $R^6$ |
| 214 | $4\text{-}OC_2H_5$ | 3-F | H | Cl | $CH_3$ | $R^6$ |
| 215 | $4\text{-}OC_2H_5$ | 3-F | H | F | $CH_3$ | $R^6$ |
| 216 | $4\text{-}OC_2H_5$ | 3-F | H | H | CN | $R^3$ |
| 217 | $4\text{-}OC_2H_5$ | 3-F | H | Cl | CN | $R^3$ |
| 218 | $4\text{-}OC_2H_5$ | 3-F | H | F | CN | $R^3$ |
| 219 | $4\text{-}OC_2H_5$ | 3-F | H | H | $CH_3$ | $R^3$ |
| 220 | $4\text{-}OC_2H_5$ | 3-F | H | Cl | $CH_3$ | $R^3$ |
| 221 | $4\text{-}OC_2H_5$ | 3-F | H | F | $CH_3$ | $R^3$ |
| 222 | 4-Cl | H | H | H | CN | $R^1$ |
| 223 | 4-Cl | H | H | Cl | CN | $R^1$ |
| 224 | 4-Cl | H | H | F | CN | $R^1$ |
| 225 | 4-Cl | H | H | H | $CH_3$ | $R^1$ |
| 226 | 4-Cl | H | H | Cl | $CH_3$ | $R^1$ |

TABLE I CONTINUED

| Compound No | $W^1$ | $W^2$ | $W^3$ | X | R | Y |
|---|---|---|---|---|---|---|
| 227 | 4-Cl | H | H | F | $CH_3$ | $R^1$ |
| 228 | 4-Cl | H | H | H | CN | $R^2$ |
| 229 | 4-Cl | H | H | Cl | CN | $R^2$ |
| 230 | 4-Cl | H | H | F | CN | $R^2$ |
| 231 | 4-Cl | H | H | H | $CH_3$ | $R^2$ |
| 232 | 4-Cl | H | H | Cl | $CH_3$ | $R^2$ |
| 233 | 4-Cl | H | H | F | $CH_3$ | $R^2$ |
| 234 | 4-Cl | H | H | H | CN | $R^6$ |
| 235 | 4-Cl | H | H | Cl | CN | $R^6$ |
| 236 | 4-Cl | H | H | F | CN | $R^6$ |
| 237 | 4-Cl | H | H | H | $CH_3$ | $R^6$ |
| 238 | 4-Cl | H | H | Cl | $CH_3$ | $R^6$ |
| 239 | 4-Cl | H | H | F | $CH_3$ | $R^6$ |
| 240 | 4-Cl | H | H | H | CN | $R^3$ |
| 241 | 4-Cl | H | H | Cl | CN | $R^3$ |
| 242 | 4-Cl | H | H | F | CN | $R^3$ |
| 243 | 4-Cl | H | H | H | $CH_3$ | $R^3$ |
| 244 | 4-Cl | H | H | Cl | $CH_3$ | $R^3$ |
| 245 | 4-Cl | H | H | F | $CH_3$ | $R^3$ |
| 246 | 4-$CH_2CH_2CH_3$ | H | H | H | CN | $R^1$ |
| 247 | 4-$CH_2CH_2CH_3$ | H | H | Cl | CN | $R^1$ |
| 248 | 4-$CH_2CH_2CH_3$ | H | H | F | CN | $R^1$ |
| 249 | 4-$CH_2CH_2CH_3$ | H | H | H | $CH_3$ | $R^1$ |
| 250 | 4-$CH_2CH_2CH_3$ | H | H | Cl | $CH_3$ | $R^1$ |
| 251 | 4-$CH_2CH_2CH_3$ | H | H | F | $CH_3$ | $R^1$ |

TABLE I CONTINUED

| Compound No | $W^1$ | $W^2$ | $W^3$ | X | R | Y |
|---|---|---|---|---|---|---|
| 252 | $4-CH_2CH_2CH_3$ | H | H | H | CN | $R^2$ |
| 253 | $4-CH_2CH_2CH_3$ | H | H | Cl | CN | $R^2$ |
| 254 | $4-CH_2CH_2CH_3$ | H | H | F | CN | $R^2$ |
| 255 | $4-CH_2CH_2CH_3$ | H | H | H | $CH_3$ | $R^2$ |
| 256 | $4-CH_2CH_2CH_3$ | H | H | Cl | $CH_3$ | $R^2$ |
| 257 | $4-CH_2CH_2CH_3$ | H | H | F | $CH_3$ | $R^2$ |
| 258 | $4-CH_2CH_2CH_3$ | H | H | H | CN | $R^6$ |
| 259 | $4-CH_2CH_2CH_3$ | H | H | Cl | CN | $R^6$ |
| 260 | $4-CH_2CH_2CH_3$ | H | H | F | CN | $R^6$ |
| 261 | $4-CH_2CH_2CH_3$ | H | H | H | $CH_3$ | $R^6$ |
| 262 | $4-CH_2CH_2CH_3$ | H | H | Cl | $CH_3$ | $R^6$ |
| 263 | $4-CH_2CH_2CH_3$ | H | H | F | $CH_3$ | $R^6$ |
| 264 | $4-CH_2CH_2CH_3$ | H | H | H | CN | $R^3$ |
| 265 | $4-CH_2CH_2CH_3$ | H | H | Cl | CN | $R^3$ |
| 266 | $4-CH_2CH_2CH_3$ | H | H | F | CN | $R^3$ |
| 267 | $4-CH_2CH_2CH_3$ | H | H | H | $CH_3$ | $R^3$ |
| 268 | $4-CH_2CH_2CH_3$ | H | H | Cl | $CH_3$ | $R^3$ |
| 269 | $4-CH_2CH_2CH_3$ | H | H | F | $CH_3$ | $R^3$ |
| 270 | $4-OCH_2CH_3$ | 3-F | 5-F | H | CN | $R^1$ |
| 271 | $4-OCH_2CH_3$ | 3-F | 5-F | H | CN | $R^3$ |
| 272 | $4-OCH_2CH_3$ | 3-F | 5-F | H | CN | $R^2$ |
| 273 | $4-OCF_3$ | 3-F | 5-F | H | CN | $R^1$ |
| 274 | $4-OCF_3$ | 3-F | 5-F | H | CN | $R^3$ |
| 275 | $4-OCF_3$ | 3-F | 5-F | H | CN | $R^2$ |

It will be appreciated that the compounds of formula I include one or two centres at which asymmetric substitution may occur. Each compound may therefore exist in a number of isomeric forms. All of the compounds listed in Table I are in the form of racemic mixtures of all possible isomer combinations, but it is to be understood that the invention includes within its scope not only such racemic isomer mixtures, but also any single isomer or isomer mixture of an invention compound.

The compounds of formula I fall into several types according to the nature of the group X. These are represented below in the case wherein Z is trifluoromethyl:

$Ar-CH(CF_3)-CH_2-OCH(R)-Y$ (IA)

$Ar-CF(CF_3)-CH_2-OCH(R)-Y$ (IB)

$Ar-CCl(CF_3)-CH_2-OCH(R)-Y$ (IC)

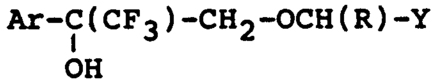
(ID)

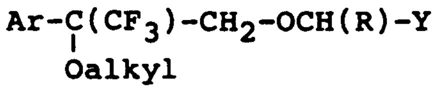
(IE)

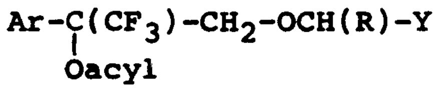
(IF)

wherein Ar represents the group of formula

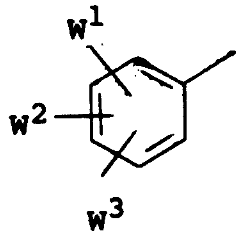

in formula I.

Compounds of formula ID may be converted to the corresponding compounds of formula IB, IC, IE and IF by reaction with an appropriate reagent, and the compounds of formula IC may be converted to the compounds of formula IA by reductive dechlorination, using for example a trialkyltin hydride as a reducing agent. Thus a compound of formula IB can be obtained by treating a compound of formula ID with a fluorinating agent such as diethylaminosulphur trifluoride (DAST), as follows :

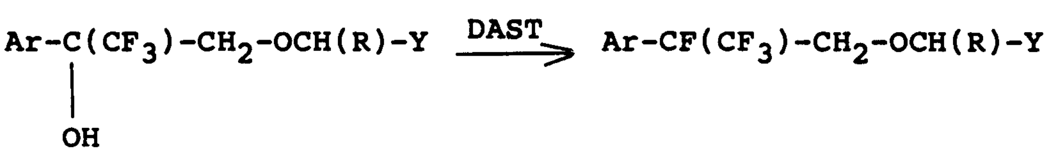


Similarly a compound of formula IC can be obtained by treating the corresponding compound of formula ID with a chlorinating agent such as thionyl chloride; a compound of fomula IG may be formed as a side produce of this reaction, presumably by dehydration of the hydroxy compound, the two products being separable by chromatographic means if required.

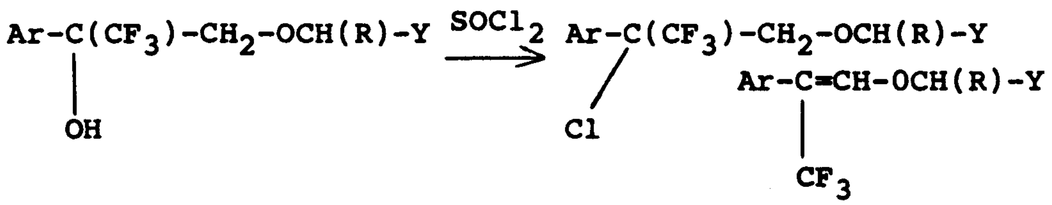

(IG)

The compounds of formula IE and IF can be obtained by conventional O-alkylation and O-acylation techniques from the corresponding compounds of formula ID. Thus reaction with a base such as sodium hydride in an aprotic solvent and an alkyl halide gives the compound of formula IE, for example:

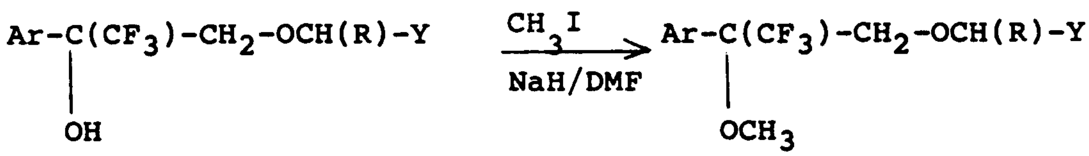


Similarly the compound of formula IF may be obtained by treating the corresponding compound of formula ID with an acyl halide or acyl anhydride in the presence of a base and a solvent, such as pyridine (py) containing a catalytic amount of 4-dimethylaminopyridine (DMAP), for example :

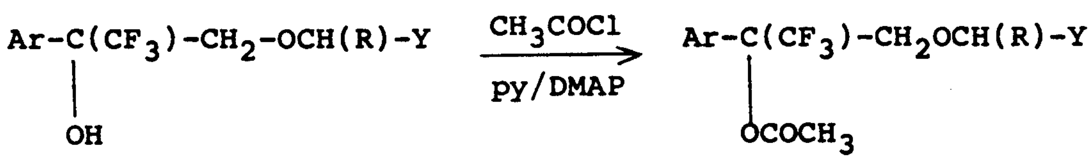


Compounds of formula ID may be prepared by processes (i), (ii) or (iii) as described below :

(i) a compound of formula :

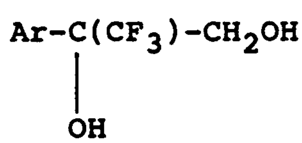
(II)

is reacted with a compound of formula Y-CH(R)-Hal, where Hal represents a halogen atom such as chlorine or bromine, optionally in the presence of a phase transfer catalyst; or

(ii) a compound of formula :

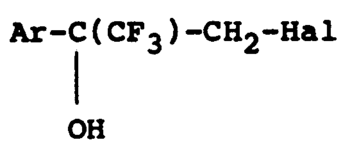
(III)

is reacted with a compound of formula Y-CH(R)-OH, where Hal represents a halogen atom such as chlorine or bromine, optionally in the presence of a phase transfer catalyst; or

(iii) a compound of formula :

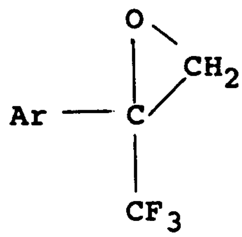

(IV)

is reacted with a compound of formula Y-CH(R)-OH in the presence of a base, such as sodium hydride and an aprotic solvent such as dimethylformamide.

The compound of formula Y-CH(R)-OH wherein Y represents 3-phenoxyphenyl and R represents trifluoromethyl may be prepared according to the method described in UK Patent No. 1,561,575.

Processes for the preparation of compounds of formula II, III and IV are outlined in the following scheme :

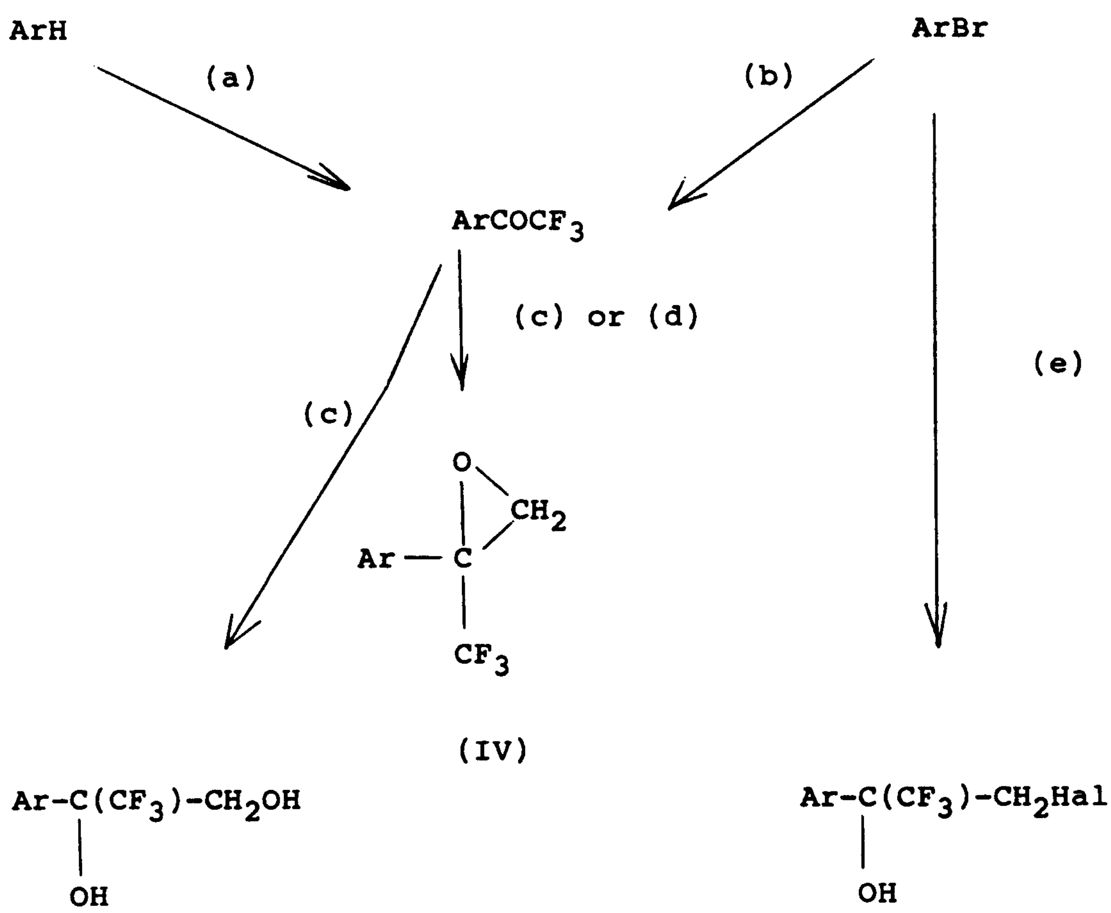


(IV)

(II)

(III)

(a) $CF_3COCl/AlCl_3$
(b) Grignard reaction using $CF_3CO_2H$ or $(CF_3CO)_2O$
(c) $[(CH_3)_3SO]^+.I^-$/KOH/t-BuOH
(d) $[(CH_3)_3SO]^+.I^-$/NaH/DMF
(e) Grignard reaction using $CF_3COCH_2Hal$

These processes are analogous to those described for the preparation of fluorinated acetophenones and corresponding derivatives of formulae II, III and IV in UK Patent Application No. 2178739.

The compounds of formula IA may also be prepared by reacting an alcohol of Formula :

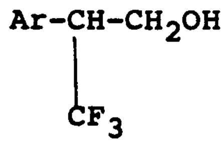

(V)

with a benzyl derivative of formula Y-CH(R)-Hal where Hal represents chloro or bromo, or a benzyl derivative of formula Y-CH(R)-Q where Q represents a displaceable group such as $-O-SO_2-CH_3$ -(methanesulphonate) or $-O-SO_2-C_6H_4-CH_3$ (p-toluenesulphonate). The reaction preferably takes place in the presence of a base such as, for example, aqueous sodium hydroxide solution, and optionally in a two phase system in the presence of a quaternary ammonium salt which functions as a phase transfer catalyst.

The alcohols of formula V may be prepared by reduction of the acids of Formula :

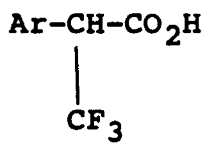

(VI)

in the form of an ester thereof, eg, a lower alkyl ester such as the ethyl ester. Suitable reducing agents for the esters include aluminium hydrides such as lithium aluminium hydride or diisobutylaluminium hydride (DIBAL), but the actual choice is dependent upon the robustness of the substituents in the group Ar in the presence of the particular reducing agent used.

The acids of formula VI may be prepared by various procedures. One process (which may be generally applicable with appropriate variation), which is illustrated in detail in the Examples hereinafter, is set out in outline in the following Scheme :

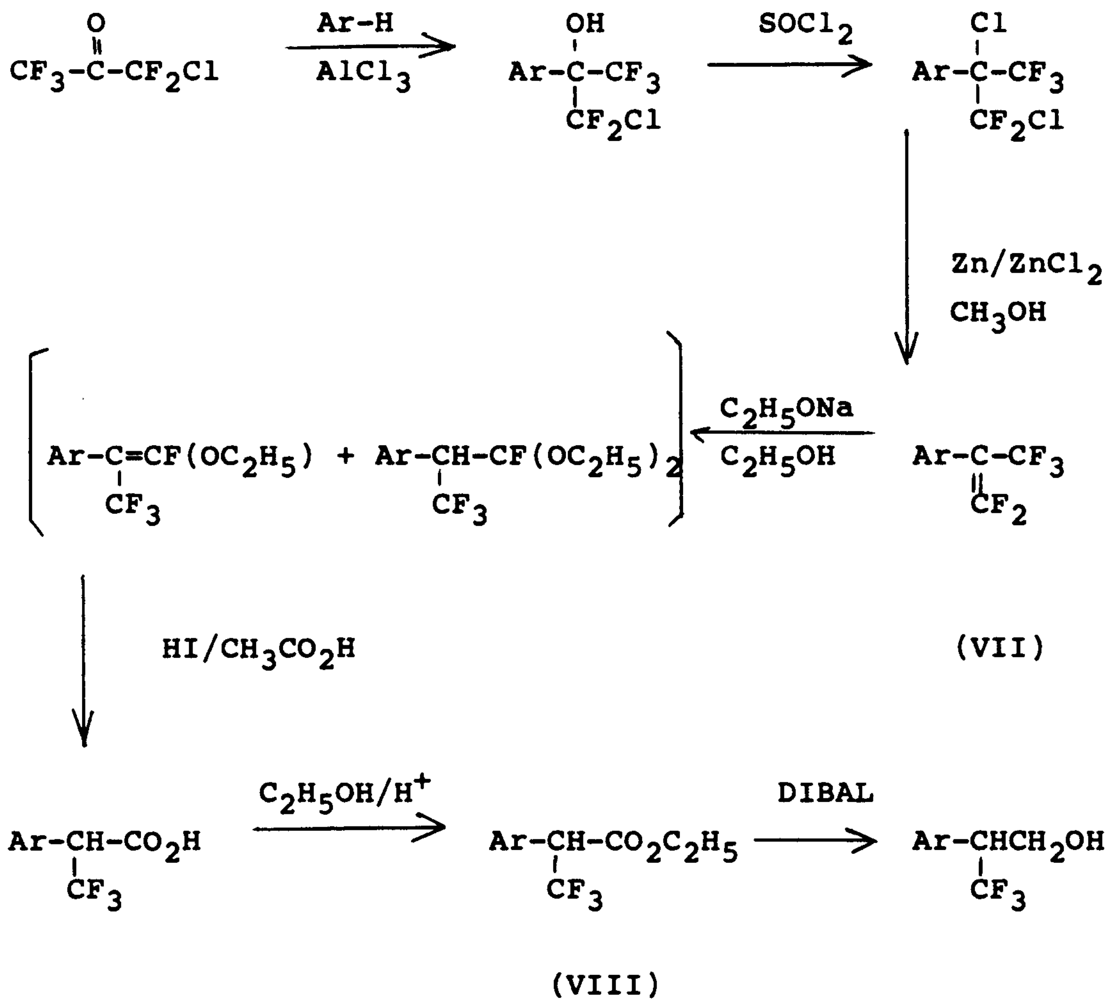

(VII)

(VIII)

The compound of Formula (VII) may also be obtained by reacting a ketone of Formula :

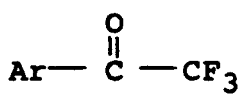

with chlorodifluoroacetic acid (in the form of a salt, eg, the sodium salt) and triphenylphosphine, by a procedure similar to that shown in J.Org.Chem. (1967), vol.32, page 1311. The esters of Formula VIII may also be prepared by reacting an aryl substituted malonate with a bromofluoromethane by a process similar to that shown in Tetrahedron Letters (1984), vol.25, page 1329:

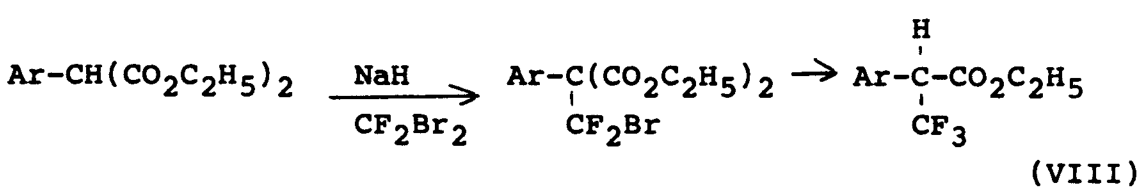

(VIII)

Alternatively the alcohols of formula V may be prepared by direct reduction of the corresponding epoxides of formula IV using, for example, catalytic hydrogenation or boranetetrahydrofuran/boron trifluoride-diethyl

ether complex, thus :

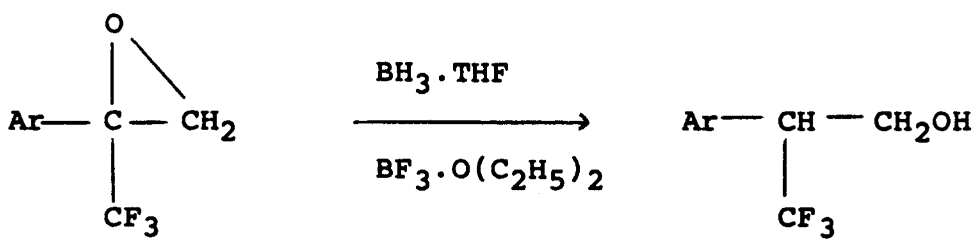


Those compounds of formulae II, III and IV wherein each of $W^1$, $W^2$ and $W^3$ represents a substituent other than hydrogen are believed not to have been described before. In a further aspect therefore the invention provides each of the compounds of formulae :

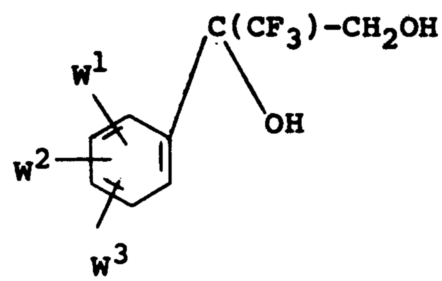

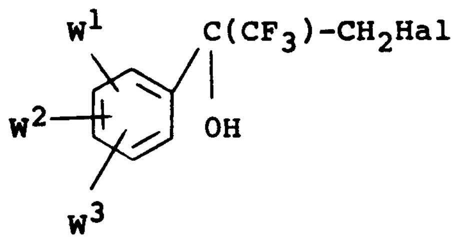

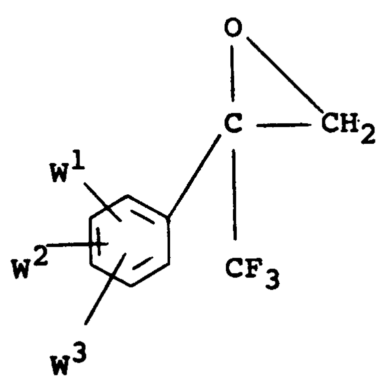

wherein $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms, and Hal represents a halogen atom.

Additionally the compounds of formulae V and VI wherein each of $W^1$, $W^2$ and $W^3$ represents a substituent other than hydrogen have not been previously described. In a yet further aspect therefore the invention provides compounds of formulae :

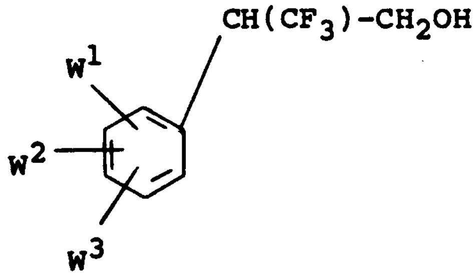

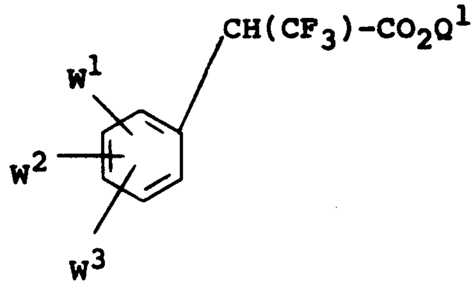

wherein $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms, and haloalkoxy of up to six carbon atoms, and $Q^1$ represents hydrogen or alkyl of up to four carbon atoms, such as methyl or ethyl.

Compounds of formula IA wherein R is a cyano group may additionally be prepared from the corresponding compounds of formula IA wherein R represents hydrogen (which may themselves be prepared by methods analogous to any of those described hereinabove for alternative values of R) by $\alpha$ -bromination using, for example, N-bromosuccinimide (NBS) in the presence of a radical initiator such as $\alpha$, $\alpha'$-Azo-bis-isobutyronitrile (AIBN), followed by displacement of the bromine atom using, for example, cuprous cyanide:

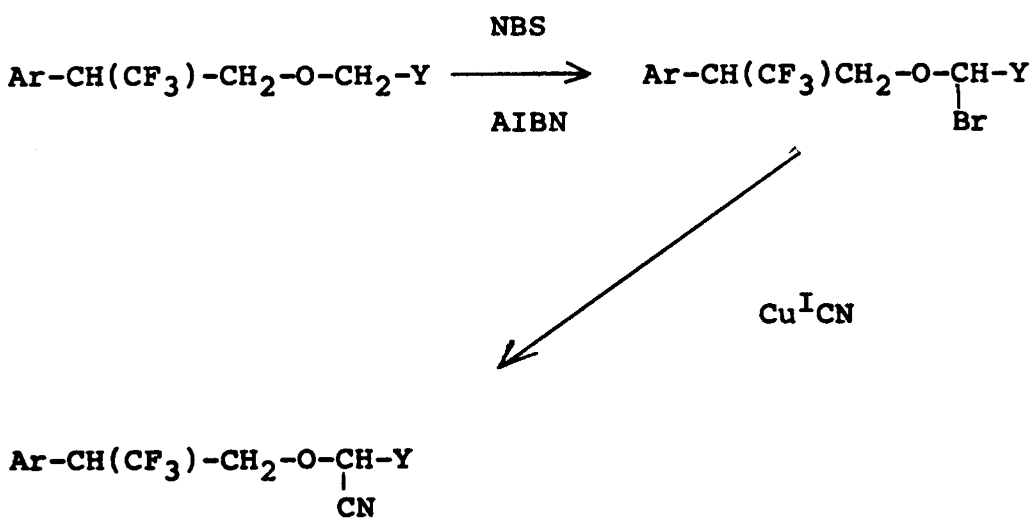


A further, generally applicable process for the preparation of compounds of formula IA is fully described in the Applicant's copending UK patent application number 8702717, and is summarised in the following scheme :

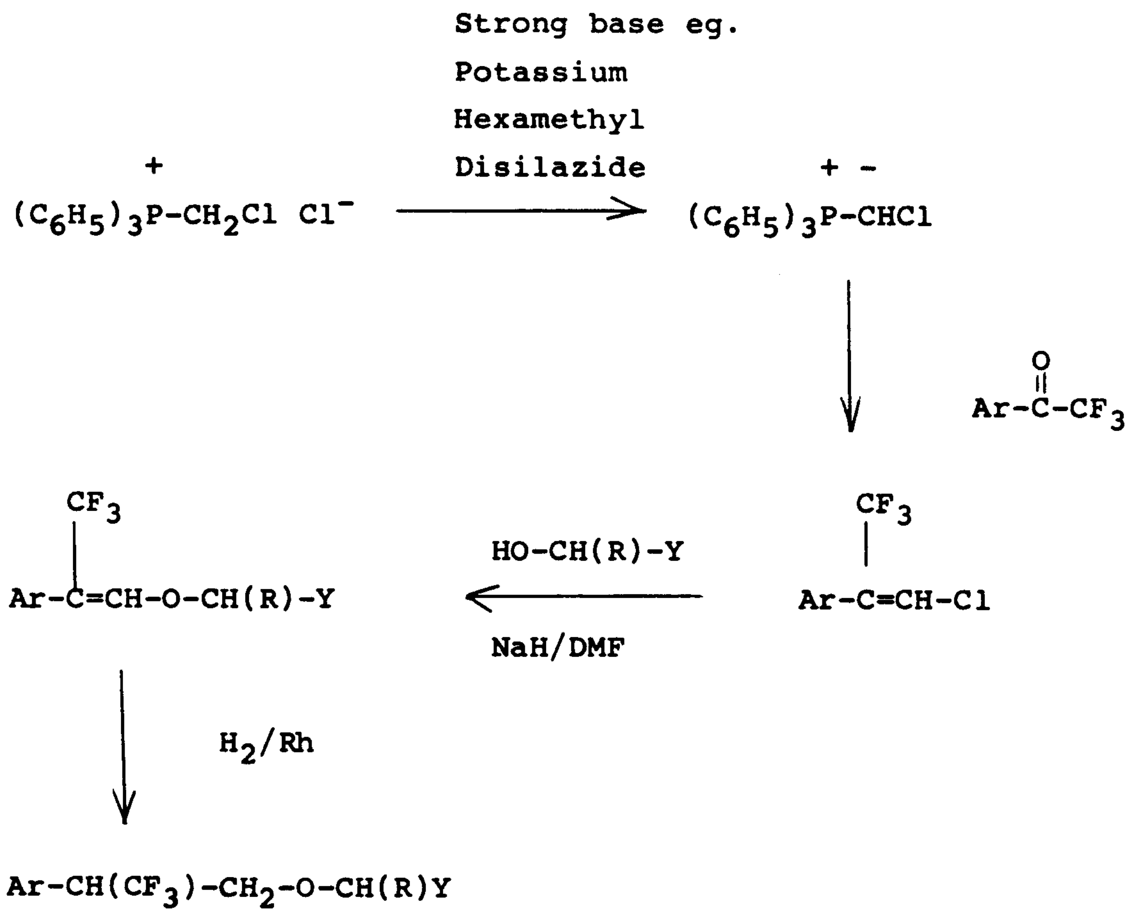


**Key : DMF = Dimethylformamide**

Further details concerning the preparation and characterisation of the compounds of the invention are given hereinafter in the Examples.

The compounds of formula (I) may be used to combat and control infestations of insect and acarine pests. The insect and acarine pests which may be combated and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula (I) suitable inert diluent or carrier materials, and/or surface active agents.

The compounds of the invention may be the sole active ingredient of the composition or they may be admixed with one or more additional active ingredients such as insecticides, insecticide synergist, herbicides, fungicides or plant growth regulators where appropriate.

Suitable additional active ingredients for inclusion in admixture with the compounds of the invention may be compounds which will broaden the spectrum of activity of the compounds of the invention or increase their persistence in the location of the pest. They may synergise the activity of the compounds of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

(a) Pyrethroids such as permethrin, esfenvalerate, deltamethrin, cyhalothrin, biphenthrin, fenpropathrin,

cyfluthrin, tefluthrin, fish safe pyrethroids for example ethofenprox, natural pyrethrins, tetramethrin, s-bioallethrin, fenfluthrin, prallethrin and 5-benzyl-3-furylmethyl-(E)-(1R,3S)-2,2-dimethyl-3-(2-oxothiolan-3-ylidenemethyl)cyclopropane carboxylate;

(b) Organophosphates such as profenofos, sulprofos, dichlorvos, methyl parathion, azinphos-methyl, demeton-s-methyl, heptenophos, thiometon, fenamiphos, monocrotophos, profenophos, triazophos, methamidophos, dimethoate, phosphamidon, malathion, chlorpyrifos, phosalone, fensulfothion, fonofos, phorate, phoxim, pyrimiphos-methyl, fenitrothion and diazinon;

(c) Carbamates (including aryl carbamates) such as pirimicarb, cloethocarb, carbofuran, ethiofencarb, aldicarb, thiofurox, carbosulfan, bendiocarb, fenobucarb, propoxur and oxamyl;

(d) Benzoyl ureas such as triflumuron, chlorofluazuron;

(e) Organic tin compounds such as cyhexatin, fenbutatin oxide, azocyclotin;

(f) Macrolides such as avermectins or milbemycins, for example such as abamectin, avermectin, and milbemycin;

(g) Hormones and synthetic mimics thereof such as juvenile hormone, juvabione, ecdysones, methoprene and hydroprene.

(h) Pheromones.

(i) Organochlorine compounds such as benzene hexachloride, DDT, chlordane or dieldrin.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin, can be employed. Alternatively insecticides or acaricides specific for particular insect species/stages for example ovolarvicides such as clofentezine, amitraz, chlordimeform, flubenzimine, hexythiazox and tetradifon, motilicides such as dicofol or propargite, adulticides such as bromopropylate, chlorobenzilate, or insect growth regulators such as hydramethylon, cyromazine, methoprene, chlorofluazuron and diflubenzuron may also be included in the compositions.

Examples of suitable insecticide synergists for use in the compositions include piperonyl butoxide, sesamex, and dodecyl imidazole.

Suitable herbicides, fungicides and plant growth regulators for inclusion in the compositions will depend upon the intended target and the effect required. An example of a rice selective herbicide which can be included is propanil, an example of a plant growth regulator for use in cotton is "Pix", and examples of fungicides for use in rice include blasticides such as blasticidin-S. The choice of other ingredients to be used in mixture with the active ingredient will often be within the normal skill of the formulator, and will be made from known alternatives depending upon the total effect to be achieved.

The ratio of the compound of the invention to any other active ingredient in the composition will depend upon a number of factors including the type of insect pests to be controlled, and the effects required from the mixture. However in general, the additional active ingredient of the composition will be applied at about the rate it would usually be employed if used on its own, or at a lower rate if synergism occurs.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material, for example pumice.

Alternatively the compositions may be in the form of liquid preparations to be used as dips, sprays or aerosols. Dips and sprays are generally aqueous dispersions or emulsions of the active ingredient in the presence of one or more known wetting agents, dispersing agents or emulsifying agents (surface active agents). Aerosol compositions may contain the active ingredient or ingredients, a propellant and an inert diluent, for example odourless kerosene or alkylated benzenes. In a preferred form, aerosol compositions may contain from 0.005% to 4% of active ingredient or ingredients, the remainder of the composition comprising a solvent, selected from odourless kerosine and alkylated benzenes, and a propellant. Aerosol compositions may optionally incorporate other additives, for example perfumes or corrosion inhibitors.

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said

partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and optionally adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 1-99% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of formula (I) and compositions comprising them are very toxic to wide varieties of insect, acarine and other invertebrate pests, including, for example, the following:

Myzus persicae (aphids)
Aphis fabae (aphids)
Megoura viceae (aphids)
Aedes aegypti (mosquitos)
Anopheles spp. (mosquitos)
Culex spp. (mosquitos)
Dysdercus fasciatus (capsids)
Musca domestica (houseflies)
Pieris brassicae (white butterfly, larvae)
Plutella maculipennis (diamond back moth, larvae)
Phaedon cochleariae (mustard beetle)
Aonidiella spp. (scale insects)
Trialeuroides spp. (white flies)
Blattella germanica (cockroaches)
Periplaneta americana (cockroaches)
Blatta orientalis (cockroaches)
Spodoptera littoralis (cotton leaf worm)
Heliothis virescens (tobacco budworms)
Chortiocetes terminifera (locusts)
Diabrotica spp. (rootworms)
Agrotis spp. (cutworms)
Chilo partellus (maize stem borers)
Nilaparvata lugens (plant hoppers)
Nephotettix cincticeps (leaf hoppers)
Panonychus ulmi
Panonychus citri
Tetranychus urticae (red spider mite)
Tetranychus cinnabarinus (carmine spider mite)

The compounds according to formula (I) and compositions comprising them have been shown to be useful for the control of lepidopteran pests, for example Spodoptera spp. and Heliothis spp. and public health pests such as flies, mosquitos and cockroaches. They are particularly useful for the control of acarine pests such as Tetranychus spp. and Panonychus spp. and pests of maize and rice such as Chilo spp. (stem borers), Nilaparvata spp. and Nephotettix spp. (plant and leaf hoppers). Some of the compounds are of particular value for the control of pests of rice because they show high levels of activity against these pests at rates which are not toxic to fish, thus enabling their use in paddy rice where fish are cultivated in the

paddy. They may also be useful in combating insect and acarine pests which infest domestic animals, such as Lucilia sericata, and ixodid ticks such as Boophilus spp., Ixodes spp., Amblyomma spp., Rhipicephalus spp. and Dermaceutor spp. They are effective in combating both susceptible and resistant strains of these pests in their adult, larval and intermediate stages of growth, and may be applied to the infested host animal by topical, oral or parenteral administration.

The following Examples illustrate various aspects of this invention. In the preparation Examples the products were usually identified and characterised by means of nuclear magnetic reasonance (NMR) spectroscopy and infra red (IR) spectroscopy. In each case where a product is specifically named its spectral characteristics are consistent with the assigned structure. Except where stated otherwise, exemplified compounds having one or more asymmetrically substituted carbon atoms were prepared in racemic form.

In the Examples, Gas Liquid Chromatography (GLC) retention times were determined on a Hewlett Packard 5890 Gas Chromatograph, using a Chrompak, CPSil 5CB column of 12.5M length and 0.2 mm internal diameter. Unless otherwise stated, the injection temperature was 100 °C, and a temperature gradient of 15 °C/minute employed, up to a maximum temperature of 250 °C, maintained for 4 minutes. The carrier gas was helium at a column head pressure maintained at 11 psi. Alternative injection and maximum temperatures are indicated in the Examples where appropriate.

$^1H$ Nuclear Magnetic Resonance (NMR) spectrometry was performed at a frequency of 270 MHz on a Jeol FX 270 NMR spectrometer, unless otherwise indicated. 90 MHz, 60 MHz, and 400 MHz $^1H$ NMR spectrometry were performed using Jeol FX 90Q, Jeol PMX 60SI, and Jeol GX400 spectrometers respectively.

$^{19}F$ NMR spectrometry was performed on a Jeol FX90Q spectrometer at a frequency of 84.26 MHz. All NMR shift ( $\delta$ ) values are quoted in ppm relative to a standard (TMS or $CFCl_3$).

Molecular Ion ($M^+$) peaks were determined on one of three mass spectrometers : Jeol DX303, Kratos MS80 or Hewlett Packard HP 5992.

The following Examples illustrates various aspects of the invention.

EXAMPLE 1

This Example illustrates the stages in the preparation of 3,5-difluoro-4-ethoxy-$\alpha,\alpha,\alpha$-trifluoroacetophenone.

Stage 1 - preparation of 4-bromo-2,6-difluorophenol.

A solution of bromine (1.6 g) in dry carbon disulphide (10 $cm^3$) was added over 5 minutes to a solution of 2,6-difluorophenol (1.3 g) in dry carbon disulphide (10 $cm^3$). To the stirred reaction mixture was added 5 drops of 48% hydrogen bromide solution. The mixture was heated for 2 hours at the reflux temperature, then allowed to stand at the ambient temperature (ca 22 °C) for 16 hours. After a further period of heating (4 hours) the mixture was allowed to stand for 24 hours before being poured into water (20 $cm^3$). To the mixture was added saturated sodium metabisulphite solution (30 $cm^3$). The layers were separated, and the organic phase was washed with saturated sodium hydrogen carbonate solution (20 $cm^3$) and water (20 $cm^3$). The organic layer was dried over anhydrous sodium sulphate then evaporated under reduced pressure to give an oil which solidified. The crude product was distilled in a Kugelrohr apparatus at an oven temperature of 100 °C under reduced pressure (ca 20 mm Hg). The distillate crystallised to give 4-bromo-2,6-difluorophenol as a white solid (0.48 g).

$^1H$ NMR ($CDCl_3$) : ca 7.1 (2H,d); ca 5.1 ($^1H$, broad)

Stage 2 - Preparation of 4-bromo-2,6-difluorophenetole.

A solution of 4-bromo-2,6-difluorophenol (1.04 g) in dry N,N-dimethylformamide (10 $cm^3$) was added over 5 minutes to a well stirred mixture of sodium hydride (0.24 g of a 50% dispersion in oil) in dry N,N-dimethylformamide (5 $cm^3$). After 30 minutes, ethyl iodide (0.78 g) was added in one portion. After stirring the reaction mixture for 30 minutes, analysis by gas liquid chromatography appeared to show no reaction; further ethyl iodide (5 g) was therefore added, and the reaction mixture was heated at 80 °C for 15 minutes. Later analysis showed similar retention times for both starting material and product, and the reaction may already have been complete prior to the second addition of ethyl iodide. The reaction mixture was washed, poured into water, and extracted with ethyl acetate. The combined organic extracts were dried over anhydrous sodium sulphate and evaporated under reduced pressure to give a red oil. The oil was purified

by column chromatography on a silica gel support, eluting with an 8:1 parts by volume mixture of n-hexane and ethyl acetate to give 4-bromo-2,6-difluorophenetole as a colourless oil (1.02 g).

$^1$H NMR ($CDCl_3$) : 1.45 (3H,t); 4.18 (2H,q); 7.06 (2H,m).

Stage 3 - Preparation of 3,5-difluoro-4-ethoxy-$\alpha,\alpha,\alpha$-trifluoroacetophenone.

A solution of 4-bromo-2,6-difluorophenetole (17.5 g) in dry tetrahydrofuran (45 $cm^3$) was added slowly to a stirred mixture of magnesium turnings (1.79 g) in dry tetrahydrofuran (50 $cm^3$) containing a crystal of iodine under an atmosphere of nitrogen. The Grignard solution was transferred to a dropping funnel and was added over 4 minutes to a solution of freshly distilled trifluoroacetic anhydride (31 g) in dry diethyl ether (70 $cm^3$) under an atmosphere of nitrogen; the reaction mixture was cooled in an ice bath during the addition. After 5 minutes the mixture was poured into ice, and stirred for 30 minutes. The products were extracted into diethyl ether and the organic phase was washed with water and sodium bicarbonate solution then dried over anhydrous sodium sulphate and the solvent evaporated under reduced pressure.

The residual oil was purified by chromatography on a silica gel support, eluting with n-hexane containing 10% by volume ethyl acetate, to give a yellow oil (10 g). This oil was distilled under reduced pressure (ca 20 mm Hg) using a Kugelrohr apparatus to give 3,5-difluoro-4-ethoxy-$\alpha,\alpha,\alpha$,-trifluoroacetophenone as a colourless oil (6.5 g).

$^1$H nmr ($CDCl_3$) : 1.44 (3H,t); 4.44 (2H,m); ca 7.6 (2H,m)

$^{19}$F nmr ($CDCl_3$) : -73.6 ($CF_3$)
-127.6 (2F,d)

EXAMPLE 2

This Example illustrates the two-stage preparation of (RS)-1,1,1-trifluoro-2-(3,5-difluoro-4-ethoxyphenyl)-3-(3-phenoxybenzyloxy)propan-2-ol (Compound No. 14)

Stage 1 - Preparation of (RS)-1,1,1-trifluoro-2-(3,5-difluoro-4-ethoxyphenyl)prop-2-eneoxide

To a stirred slurry of trimethylsulphoxonium iodide (2.2 g) in dry dimethylformamide (10 $cm^3$) was added sodium hydride (0.48 g of a 50% dispersion in oil), under an atmosphere of nitrogen. When effervescence had subsided, a solution of 3,5-difluoro-4-ethoxy-$\alpha,\alpha,\alpha$-trifluoroacetophenone (2.54 g) in dry dimethylformamide (10 $cm^3$) was added in one portion. The reaction was monitored by gas liquid chromatography of withdrawn samples. After 5 minutes, no starting Ketone was detected in the reaction mixture. The resulting solution of (RS)-1,1,1-trifluoro-2-(3,5-difluoro-4-ethoxyphenyl)prop-2-ene oxide was used immediately in the next stage, without isolation.

Stage 2

Sodium hydride (0.48 g of a 50% dispersion in oil) was added to a stirred solution of m-phenoxybenzyl alcohol (2g in dry N,N-dimethylformamide (20 $cm^3$) under an atmosphere of dry nitrogen at the ambient temperature (ca 20°C), and the mixture was stirred for 2 hours. The solution of (RS)-1,1,1-trifluoro-2-(3,5-difluoro-4-ethoxyphenyl) prop-2-ene oxide prepared in stage 1 was then added and the reaction mixture stirred for a further 2 hours. The mixture was then poured into water (100 $cm^3$) and extracted with diethyl ether (2 × 100 $cm^3$). The combined organic layers were dried over anhydrous sodium sulphate and evaporated under reduced pressure to give an oil which was purified by chromatography on a silica gel support, eluted with a mixture of n-hexane (8 parts by volume) containing ethyl acetate (1 part by volume), to give (RS)-1,1,1-trifluoro-2-(3,5-difluoro-4-ethoxyphenyl)-3-(3-phenoxybenzyloxy)propan-2-ol (0.7 g) - approximately 90% pure by gas liquid chromatographic analysis.

$^1$H NMR ($CDCl_3$) : 1.4 (3H,t); ca 3.6 (1H,m); 3.72 (1H,s); 4.0 (1H,d); 4.23 (2H,q); 4.6 (2H,s); 6.9-7.4 (11H,m)

EXAMPLE 3

The following compounds were prepared by a procedure similar to that described in Example 2.

(i) (RS)-1,1,1-trifluoro-2-(3,5-difluoro-4-ethoxyphenyl)-3-(3-phenoxy-4-fluorobenzyloxy)propan-2-ol

(Compound No. 17)

$^1$H NMR ($CDCl_3$) : 1.4 (3H,t); 3.56 (1H,m); 3.65 (1H,s); 3.96 (1H,d); 4.25 (2H,q); 4.52 (2H,s); 6.9-7.4 (10H,m)

(ii) (RS)-1,1,1-trifluoro-2-(3,5-difluoro-4-ethoxyphenyl)-3-[3-(4-chlorophenoxy)benzyloxy]propan-2-ol (Compound No. 16)

$^1$H NMR ($CDCl_3$) : 1.39 (3H,t); 3.6 (1H,m); 3.69 (1H,s); 3.99 (1H,d); 4.21 (2H,q); 4.58 (2H,s); 6.9-7.4 (10 H,m)

EXAMPLE 4

This Example illustrates the preparation of (RS)-1,1,1-trifluoro-2-chloro-2-(3,5-difluoro-4-ethoxyphenyl)-3-(3-phenoxybenzyloxy)propane (Compound No. 21).

A solution of (RS)-1,1,1-trifluoro-2-(3,5-difluoro-4-ethoxyphenyl)-3-(3-phenoxybenzyloxy)propan-2-ol (0.65 g) and imidazole (0.57 g) in dry acetonitrile (40 $cm^3$) was cooled to 0°C by external cooling and thionyl chloride (0.3 $cm^3$) was added.

The mixture was allowed to warm to the ambient temperature (ca 20°C) and was then stirred for 3 hours. Analysis by thin layer chromatography showed no remaining starting alcohol. The reaction mixture was poured into water (150 $cm^3$) and the product was extracted into diethyl ether (3 × 50 $cm^3$). The combined organic layers were washed with saturated aqueous sodium hydrogen carbonate solution, then dried over anhydrous sodium sulphate and evaporated under reduced pressure to give a brown oil. This oil was purified by column chromatography on a silica gel support, using a mixture of n-hexane (7 parts by volume) and ethyl acetate (1 part by volume) as eluent. The first major fraction was characterised as (RS)-1,1,1-trifluoro-2-chloro-2-(3,5-difluoro-4-ethoxyphenyl)-3-(3-phenoxybenzyloxy)propane (0.2g-90% pure by gas liquid chromatography).

$^1$H NMR ($CDCl_3$) : 1.4 (3H,t); 4.0 (1H,d); 4.15 (1H,m); 4.25 (2H,q); 4.62 (2H,ABq); 6.9 - 7.4 (11H,m)

The second major fraction obtained was characterised as a mixture of the above compound (83%) and 1,1,1-trifluoro-2-(3,5-difluoro-4-ethoxyphenyl)-3-(3-phenoxybenzyloxy)prop-2-ene (10%).

EXAMPLE 5

The following compounds were prepared by a procedure similar to that described in Example 4 above.

(i) (RS)-1,1,1-Trifluoro-2-chloro-2-(3,5-difluoro-4-ethoxyphenyl)-3-(3-(4-chlorophenoxy)benzyloxy)propane (Compound No. 23)

$^1$H NMR ($CDCl_3$) : 1.4 (3H,t); 4.0 (1H,d); 4.17 (1H,d); 4.25 (2H,q); 4.61 (2H,ABq); 6.9-7.7 (10H,m).

(ii) (RS)-1,1,1-Trifluoro-2-chloro-2-(3,5-difluoro-4-ethoxyphenyl)-3-(3-phenoxy-4-fluorobenzyloxy)propane (Compound No. 24).

$^1$H NMR ($CDCl_3$) : 1.4 (3H,t); 4.0 (1H,d); 4.15 (1H,d); 4.24 (2H,q); 4.62 (2H,ABq); 6.9-7.4 (10H,m)

EXAMPLE 6

This Example illustrates the preparation of (RS)-1,1,1-trifluoro-2-(3,5-difluoro-4-ethoxyphenyl)-3-(3-phenoxybenzyloxy)propane (Compound No. 1).

(RS)-1,1,1-Trifluoro-2-chloro-2-(3,5-difluoro-4-ethoxyphenyl)-3-(3-phenoxybenzyloxy)propane (0.2 g), $\alpha,\alpha'$-azoisobutyronitrile (0.01 g) and tri-n-butyltin hydride (0.13 g) were mixed in toluene (10 $cm^3$), and the reaction mixture heated at 95°C for 8 hours. Analysis by gas liquid chromatography then showed no starting material remaining. The solvent was evaporated under reduced pressure, and the residual oil purified by chromatography on a silica gel support, eluting with n-hexane contaning 5% by volume ethyl acetate, to give 1,1,1-trifluoro-2-(3,5-difluoro-4-ethoxyphenyl)-3-(3-phenoxybenzyloxy)-propane (0.14 g) as a colourless oil (97% pure).

$^1$H NMR ($CDCl_3$) : 1.40 (3H,t); 3.5 (1H,m); 3.77 (1H,dd); 3.92 (1H,dd); 4.22 (2H,q); 4.5 (2H,ABq); 6.8 - 7.4 (11H,m)

EXAMPLE 7

The following compounds were prepared by a procedure similar to that described in Example 6.

(i) (RS)-1,1,1-Trifluoro-2-(3,5-difluoro-4-ethoxyphenyl)-3-(3-phenoxy-4-fluorobenzyloxy)propane (Compound No. 4).

$^1$H NMR ($CDCl_3$) : 1.39 (3H,t); 3.5 (1H,m); 3.75 (1H,dd); 3.9 (1H,dd); 4.22 (2H,q); 4.42 (2H,ABq);

6.8-7.4 (10H,m)

(ii) (RS)-1,1,1-Trifluoro-2-(3,5-difluoro-4-ethoxyphenyl)-3-(3-(4-chlorophenoxy)benzyloxy)propane (Compound No. 3).

1H NMR ($CDCl_3$) : 1.40 (3H,t); 3.5 (1H,m); 3.77 (1H,dd); 3.93 (1H,dd); 4.22 (2H,q); 4.47 (2H,ABq); 6.85-7.0 (7H,m); ca 7.3 (3H,m)

EXAMPLE 8

This Example illustrates the preparation of (RS)-1,1,1-trifluoro-2-(4-ethoxyphenyl)prop-2-ene oxide.

Trimethylsulphoxonium iodide (8.1 g) was added to a stirred solution of 4-ethoxy- α,α,α-trifluoroacetophenone (8g, prepared according to the method described in Example 3 of UK Patent Application No. 2178739) in t-butanol (25 $cm^3$). When the addition was complete, potassium hydroxide pellets (2g) were added, and the reaction mixture was heated at the reflux temperature for one hour. The mixture was cooled, and poured into a dilute aqueous solution of hydrochloric acid. The aqueous mixture was extracted eight times with diethyl ether and the combined extracts were dried over anhydrous magnesium sulphate. Removal of the solvent by evaporation under reduced pressure gave a pale yellow oil (5.5g) containing a small amount of solid residue. The crude product residue was passed through a plug of silica gel, using n-hexane containing 10% by volume ethyl acetate as eluent. Further purification by chromatography using a silica gel column eluted with n-hexane containing 10% by volume ethyl acetate gave two fractions containing (RS)-1,1,1-trifluoro-2-(4-ethoxyphenyl)prop-2-ene oxide. The first fraction was shown by gas liquid chromatography to be 79% pure, the second 98% pure.

$^1H$ NMR ($CDCl_3$) : 1.42 (3H,t); 2.9 (1H,dq); 3.38 (1H,d); 4.07 (2H,q); 6.9-7.4 (4H,m).

EXAMPLE 9

This Example illustrates the preparation of (RS)-1,1,1-trifluoro-2-(4-ethoxyphenyl)propan-3-ol.

A solution of boron trifluoride etherate (0.12 $cm^3$) in tetrahydrofuran (2 $cm^3$) was added to a one molar (1M) solution of borane-tetrahydroforan (0.9 $cm^3$) tetrahydrofuran (1 $cm^3$) under an atmosphere of nitrogen. The temperature of the mixture was cooled to 0 °C by external cooling, and a solution of (RS)-1,1,1-trifluoro-2-(4-ethoxyphenyl)prop-2-ene oxide (0.1g) in tetrahydrofuran (1 $cm^3$) was added with stirring. The mixture was allowed to warm to the ambient temperature (ca. 22 °C) and stirred overnight. The mixture was then partitioned between saturated potassium carbonate solution (6 $cm^3$) and diethyl ether (10 $cm^3$). The aqueous layer was separated and extracted twice with diethyl ether (10 $cm^3$). The combined organic layers were dried over anhydrous sodium sulphate and the solvent evaporated under reduced pressure to give a pale yellow oil (0.09g). This oil was purified by column chromatography on a silica gel support, eluting with n-hexane containing 30% diethyl ether, to give (RS)-1,1,1-trifluoro-2-(4-ethoxyphenyl)propan-3-ol (0.05g) as a colourless oil.

$^1H$ NMR ($CDCl_3$) : 1.4 (3H,t); 1.6 (1H,broad s); 3.5 (1H,m); 4.0 (2H,q) overlapping with (2H,m); 7.0 (2H,d); 7.2 (2H,d).

IR (liquid film) : 3400 (broad), 1620, 1520, 1310, 1255, 1166, 1120, 1050 $cm^{-1}$

GLC retention time : 6.06 minutes (50 °C-280 °C run)

EXAMPLE 10

This Example illustrates the preparation of (2RS)-1,1,1-trifluoro-2-(4-ethoxyphenyl)-3-[(1RS)-1-(3-phenoxyphenyl)ethoxy]propane (Compound No. 94).

A solution of (RS)-1-(3-phenoxyphenyl)ethanol (0.25g) in dichloromethane (5 $cm^3$) was cooled to 0 °C and triethylamine (0.205g) was added followed by mesyl chloride (0.165g). The reaction mixture was stirred for 1 hour at the ambient temperature before being diluted with diethyl ether (5 $cm^3$) and filtered. Evaporation of the solvent under reduced pressure gave the mesylate derivative, which was used directly without purification. The mesylate (0.19g) was dissolved in dichloromethane (3 $cm^3$) and treated sequentially with 1,1,1-trifluoro-2-(4-ethoxyphenyl)propan-3-ol (0.08g), tetra-n-butylammonium hydrogen sulphate (0.015g) and 40% aqueous sodium hydroxide solution.

The reaction mixture was stirred overnight, then separated. The organic phase was washed with water (8 $cm^3$) and dried over anhydrous sodium sulphate. Evaporation of the solvent under reduced pressure gave an oil which was purified by column chromatography to give (2RS)-1,1,1-trifluoro-2-(4-ethoxyphenyl)-3-[-(1RS)-1-(3-phenoxyphenyl)ethoxy]propane (0.125g) as a colourless oil.

$^1H$ NMR ($CDCl_3$) : 1.2 (6H,m); 3.5 (1H,m); 3.6 (1H,m); 3.8 (1H,m); 4.0 (2H, overlapping 2q); 4.3

(1H,m); 6.8-7.35 (13H,m).
GLC retention times: 10.48 minutes (50%) 10.64 minutes (50%) (separation due to pairs of diastereoisomers)

EXAMPLE 11

This Example illustrates the preparation of (RS)-1,1,1-trifluoro-2-(4-ethoxyphenyl)-3-(3-phenoxybenzyloxy)propane from (RS)-1,1,1-trifluoro-2-(4-ethoxyphenyl)propan-3-ol.

A mixture of (RS)-1,1,1-trifluoro-2-(4-ethoxyphenyl)propan-3-ol (0.4 g), 3-phenoxybenzyl bromide (0.45 g), tetra-n-butyl-ammonium hydrogen sulphate (0.05 g) and aqueous sodium hydroxide solution (40% w/v, 5.0 $cm^3$) was stirred at the ambient temperature for 6 hours after which it was partitioned between water and diethyl ether. The ethereal phase was separated, washed twice with water, dried over anhydrous magnesium sulphate and concentrated by evaporation of the solvent under reduced pressure. The residual oil (0.75 g) was purified by chromatography on a silica gel column eluted with a mixture of hexane (23 parts by volume) and ethyl acetate (2 parts by volume) to yield (RS)-1,1,1-trifluoro-2-(4-ethoxyphenyl)-3-(3-phenoxybenzyloxy)propane (0.21 g) as a viscous oil.

IR (liquid film) : 1617, 1590, 1520, 1490, 1448, 1260, 1220, 1170, 1126, 1076, 1050, 700 $cm^{-1}$
$^1$H NMR ($CDCl_3$) : 1.42 (3H,t); 3.55 (1H,q); 3.8 (1H,m); 3.95 (1H,m); 4.0 (2H,q); 4.46 (ABq,2H); 6.8-7.4 (13H,m)
$^{19}$F NMR ($CDCl_3$) : -68.43 (3F,d)
GLC retention time : 11.15 minutes.

EXAMPLE 12

This Example illustrates the stages in the preparation of (2RS)-1,1,1-trifluoro-2-(4-ethoxyphenyl)-3-[(RS)-α-cyano-3-phenoxybenzyloxy]propane (Compound No. 90).

(i) A catalytic amount of α,α′-azo-iso-butyronitrile was added to a solution of N-bromosuccinimide (0.14g) and (RS)-1,1,1-trifluoro-2-(4-ethoxyphenyl)-3-(3-phenoxybenzyloxy)propane (0.3g) in carbon tetrachloride (14 $cm^3$). The reaction mixture was heated at the reflux temperature for 90 minutes, then cooled to 0°C, filtered, and the filtrate concentrated by evaporation under reduced pressure to leave (2RS)-1,1,1-trifluoro-2-(4-ethoxyphenyl)-3-[(RS)-α-bromo-3-phenoxybenzyloxy]-propane as a pale yellow oil which was used without further purification.

(ii) The crude brominated product from stage (i) was dissolved in toluene (10 $cm^3$) and cuprous cyanide (0.3 g) added. The reaction mixture was heated at the reflux temperature for 14 hours and then stirred at the ambient temperature for 7 days. The mixture was filtered and the filtrate evaporated under reduced pressure to leave an oil, which was purified by high performance liquid chromatography to give (2RS)-1,1,1-trifluoro-2-(4-ethoxyphenyl)-3-[(RS) α-cyano-3-phenoxybenzyloxy]propane (0.025 g) as a colourless oil.

$^1$H NMR ($CDCl_3$) : 1.41 (3H,t); 3.59 (1H,m); 3.90 (1H,m); 4.0-4.2 (4H,m); 5.2-5.3 (1H,2s); 6.85-7.4 (13H,m);
GLC retention time: 11.64 minutes (50%) 11.68 minutes (50%) separation due to pairs of diastereoisomers.

EXAMPLE 13

This Example illustrates the stages in the preparation of (2RS)-1,1,1-trifluoro-2-(4-ethoxyphenyl)-3-[(RS)--trifluoromethyl-3-phenoxybenzyloxy]propane (Compound No. 102).

(i) EZ-1,1,1-trifluoro-2-(4-ethoxyphenyl)-3-chloroprop-2-ene

Potassium disilazide (76 $cm^3$ of a 1M solution in tetrahydrofuran) was added slowly to a stirred solution of (chloromethyl)triphenylphosphonium chloride (27g) in dry tetrahydrofuran (100 $cm^3$) at 0°C. The solution was stirred for 30 minutes to allow formation of the ylid, then added to a solution of α,α,α-trifluoro-4-ethoxyacetophenone (15g) in tetrahydrofuran (20 $cm^3$) at 0°C. After warming to the ambient temperature, the reaction mixture was poured into water (100 $cm^3$) and the product extracted into diethyl ether (3 × 50 $cm^3$). The combined organic layers were dried over anhydrous sodium sulphate, and the solvent evaporated. The crude residue was purified by column chromatography on a silica gel support, eluted with n-hexane containing 3% by volume diethyl ether, to give EZ-1,1,1-trifluoro-2-(4-ethoxyphenyl)-3-chloroprop-2-ene (3.5g).

$^1$H NMR ($CDCl_3$) : 1.4 (3H,t); 4.05 (2H,q); 6.9 (2H,d); 7.05 (1H,m); 7.3 (2H,d)

E:Z isomer ratio approx 1:1.

(ii) EZ-1,1,1-Trifluoro-2-(4-ethoxyphenyl)-3-[(RS)-α-trifluoromethyl-3-phenoxybenzyloxy]prop-2-ene.

Sodium hydride (0.11g of a 50% dispersion in oil) was added to a stirred solution of α-trifluoromethyl-3-phenoxybenzyl alcohol (0.6g, prepared according to the method of UK Patent Specification No. 1,561,575) in dry dimethylformamide. After effervescence had subsided, the resulting mixture was added dropwise to a stirred solution of EZ-1,1,1-trifluoro-2-(4-ethoxyphenyl)-3-chloroprop-2-ene (1.12g) in dry dimethylformamide (10 $cm^3$), the temperature of the reaction mixture being maintained at -40°C. The reaction mixture was then allowed to warm to the ambient temperature, and was stirred for 6 hours. The mixture was acidified with dilute aqueous acetic acid solution and the product extracted into diethyl ether. The combined ethereal layers were dried over anhydrous magnesium sulphate and the solvent evaporated under reduced pressure. The crude product was purified by column chromatography on a silica gel support, eluting with dichloromethane containing 3% by volume hexane.

$^1H$ NMR ($CDCl_3$) : 1.4 (3H,t); 4.0 (2H,q); 5.05 (1H,q); 6.8-7.4 (14H,m)

(iii) (2RS)-1,1,1-trifluoro-2-(4-ethoxyphenyl)-3-[(RS)-α-trifluoromethyl-3-phenoxybenzyloxy)]propane (Compound No. 102).

A solution of EZ-1,1,1-trifluoro-2-(4-ethoxyphenyl)-3-[(RS)-α-trifluoromethyl-3-phenoxybenzyloxy]-prop-2-ene (0.37g) in ethanol (25 $cm^3$) containing 5% Rhodium on Aluminia catalyst (0.06g) was hydrogenated at 3-5 atmospheres for 4 hours. The mixture was filtered to remove the catalyst and the solvent evaporated under reduced pressure. Purification of the crude product by column chromatography on a silica gel support, eluting with hexane containing 33% by volume toluene, followed by a second purification on a silica column eluting with hexane containing 10% by volume ethyl acetate, gave the title compound as a mixture of diastereoisomers.

$^1H$ NMR ($CDCl_3$) : 1.4 (3H,2t); 3.55 (1H,m); 3.8 (1H,m); 4.0 (3H,m); 4.5 (1H,2q); 6.8-7.4 (13H,m)

## EXAMPLE 14

This Example illustrates the insecticidal and acaricidal properties of the Products of this invention.

The activity of the Product was determined using a variety of insect and acarine pests. The Product was used in the form of liquid preparations containing 500 parts per million (ppm) by weight of the Product. The preparations were made by dissolving the Product in acetone and diluting the solutions with water containing 0.01% by weight of a wetting agent sold under the trade name "LISSAPOL" NX until the liquid preparations contained the required concentration of the Product. "Lissapol" is a Registered Trade Mark.

The test procedure adopted with regard to each pest was basically the same and comprised. supporting a number of the pests on a medium which was usually a host plant or a foodstuff on which the pests feed, and treating either or both the pests and the medium with the preparations. The mortality of the pests was then assessed at periods usually varying from one to three days after the treatment.

In the case of the species Musca domestica (housefly), additional tests to determine the knockdown effect of the compounds were performed. Details are given in Table II.

The results of the tests are given in Table III for each of the Products, at the rate in parts per million given in the second column as a grading of mortality designated as A, B or C wherein A indicates 80-100% mortality or knockdown (70-100% in the case of Spodoptera exigua), B indicates 50-79% mortality or knockdown (50-69% in the case of Spodoptera exigua) and C indicates less than 50% mortality or knockdown.

In Table III the pest organism used is designated by a letter code and the pest species, the support medium or food, and the type and duration of test is given in Table II.

TABLE II

| CODE LETTERS (Table IV) | TEST SPECIES | SUPPORT MEDIUM/FOOD | TYPE OF TEST | DURATION (days) |
|---|---|---|---|---|
| TU | *Tetranychus urticae* (spider mites - adult) | French bean leaf | Contact | 3 |
| MP | *Myzus persicae* (aphids) | Chinese Cabbage leaf | Contact | 3 |
| NL | *Nilaparvata lugens* (green leaf hopper) | Cabbage leaf | Contact | 3 |
| HV | *Heliothis virescens* (tobacco budworm) | Cotton leaf | Residual | 3 |
| DB | *Diabrotica balteata* (rootworm larvae) | Filter paper/ maize seed | Residual | 3 |
| BG | *Blattella germanica* (cockroach nymphs) | Plastic pot | Residual | 3 |
| MD | *Musca domestica* (houseflies - adults) | Cotton wool/ sugar | Contact | 1 |
| MD/KD | *Musca domestica* (houseflies - adults) | Cotton/wool sugar | Knockdown | 4 hours |
| SE | *Spodoptera exigua* (lesser armyworm) | Cotton leaf | Residual | 3 |

"Contact" test indicates that both pests and medium were treated and "residual" indicates that the medium was treated before infestation with the pests.

TABLE III

| Compound No. | Rate (ppm) | TU | MP | NL | MD/ KD | MD | BG | HV | SE | DB |
|---|---|---|---|---|---|---|---|---|---|---|
| 1 | 500 | A | A | A | A | A | A | A | A | A |
| 3 | 500 | A | A | A | A | A | A | A | A | A |
| 4 | 500 | A | B | A | A | A | A | A | A | A |
| 14 | 500 | C | C | C | A | C | C | B | A | A |
| 90 | 500 | C | A | A | A | A | B | A | A | A |
| 94 | 500 | C | A | A | B | A | C | A | C | C |
| 102 | 500 | B | C | - | C | C | C | C | A | C |

## Claims

**1.** A compound of formula :

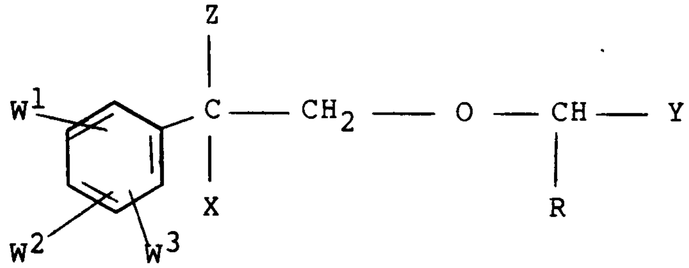

wherein X is selected from hydrogen, halo, hydroxy, alkoxy of up to four carbon atoms and acyloxy of up to four carbon atoms, Z represents a fluoroalkyl group of one or two carbon atoms, Y represents a substituted aryl group where each substituent is selected from halo, alkyl of up to six carbon atoms, aryl, aralkyl of up to four carbon atoms in the alkyl moiety, aryloxy and arylamino, and either

(i) R represents hydrogen, and $W^1$, $W^2$ and $W^3$ are independently selected from halo, alkyl of up to six carbon atoms, alkoxy of up to six carbon atoms, alkoxyalkyl of up to a total of six carbon atoms,

haloalkyl of up to six carbon atoms, and haloalkoxy of up to six carbon atoms, or

(ii) R is selected from methyl, trifluoromethyl, cyano and ethynyl, and $W^1$, $W^2$ and $W^3$ are independently selected from hydrogen, halo, alkyl of up to six carbon atoms, alkoxy of up to six carbon atoms, alkoxyalkyl of up to a total of six carbon atoms, haloalkyl of up to six carbon atoms, and haloalkoxy of up to six carbon atoms.

**2.** A compound as claimed in claim 1 wherein X is selected from hydrogen, halo and hydroxy, Z represents the trifluoromethyl group, Y represents an aryl group selected from phenyl, pyridyl and furyl, substituted with one or more substituents selected from fluoro, methyl, phenyl, benzyl, phenoxy chlorophenoxy, fluorophenoxy, bromophenoxy and fluoroanilino, and either

(i) R represents hydrogen, and $W^1$, $W^2$ and $W^3$ are independently selected from fluoro, chloro, bromo, alkyl of up to four carbon atoms, alkoxy of up to four carbon atoms, alkoxyalkyl of up to a total of four carbon atoms, haloalkyl of up to two carbon atoms and haloalkoxy of up to two carbon atoms, or

(ii) R is selected from methyl, trifluoromethyl, cyano and ethynyl, and $W^1$, $W^2$ and $W^3$ are independently selected from hydrogen, fluoro, chloro, bromo, alkyl of up to four carbon atoms, alkoxy of up to four carbon atoms, alkoxyalkyl of up to a total of four carbon atoms, haloalkyl of up to two carbon atoms and haloalkoxy of up to two carbon atoms.

**3.** A compound as claimed in claim 1 wherein X is selected from hydrogen, fluoro and chloro, Z represents trifluoromethyl, Y represents a phenoxyphenyl group in which each phenyl group may be unsubstituted or substituted with halogen, and R, $W^1$, $W^2$ and $W^3$ are as described in claim 1, with the proviso that one of $W^1$, $W^2$ and $W^3$ represents a substituent in the 4-position selected from chloro, fluoro, methoxy, ethoxy, trifluoromethyl, trifluoromethoxy and difluoromethoxy.

**4.** An insecticidal and acaricidal composition comprising an insecticidally and acaricidally effective amount of a compound as defined in claim 1 in association with an insecticidally and acaricidally inert diluent or carrier.

**5.** A method of combating insect and acarine pests at a locus which comprises applying to the locus an insecticidally and acaricidally effective amount of a composition as defined in claim 2.

**6.** A process for preparing a product of formula :

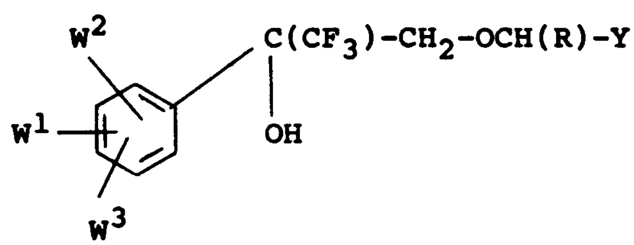

wherein $W^1$, $W^2$, $W^3$, R and Y are as defined in claim 1, which comprises either (i) reacting a compound of formula :

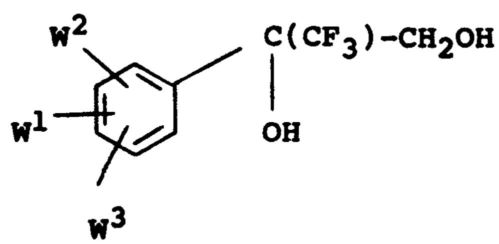

with a compound of formula :

Y-CH(R)-Hal

or (ii) reacting a compound of formula :

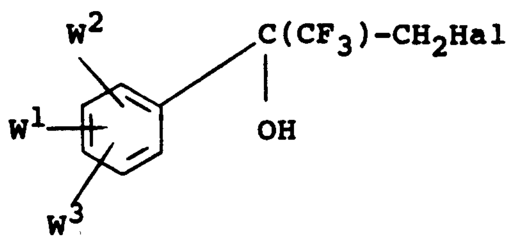

with a compound of formula :

Y-CH(R)-OH

or (iii) reacting a compound of formula :

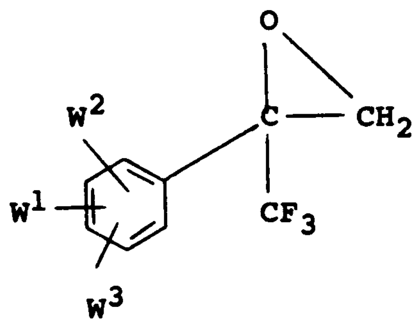

with a compound of formula :

Y-CH(R)-OH

and thereafter, if desired, converting the product
(a) to a compound of formula :

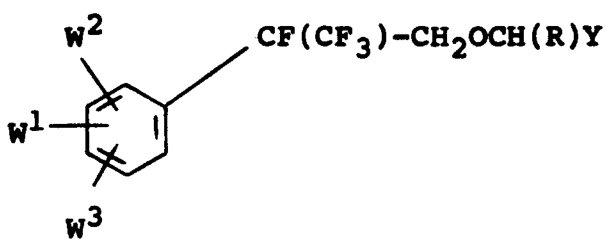

by reacting with a fluorinating agent; or
(b) to a compound of formula :

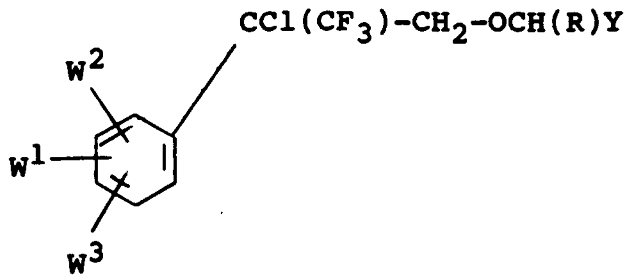

and/or a compound of formula :

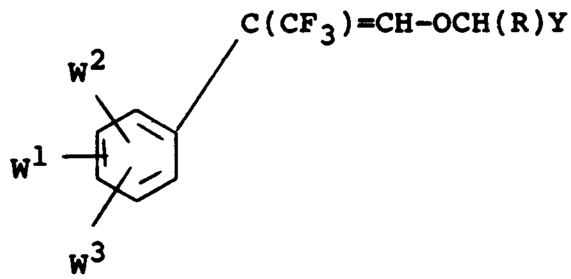

by reaction with an inorganic acid chloride, or
(c) to an O-alkyl derivative by reaction with an alkyl halide and a base, or
(d) to an O-acyl derivative by reaction with an acyl chloride or acyl anhydride,
wherein Hal represents chlorine or bromine.

**7.** A process for preparing a compound of formula :

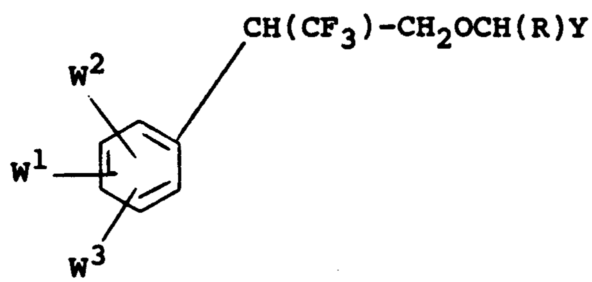

wherein $W^1$, $W^2$, $W^3$, R and Y are as defined in claim 1, which comprises reductive dechlorination of the corresponding compound of formula :

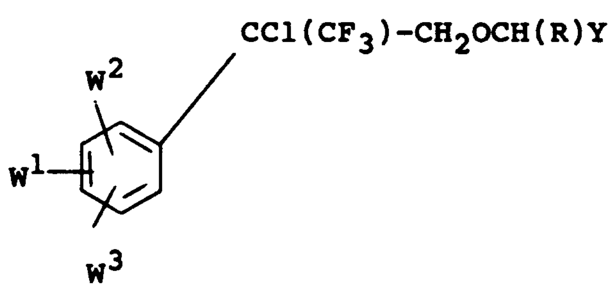

**8.** A process for preparing a compound of formula :

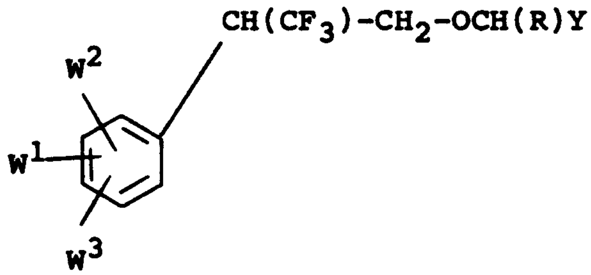

wherein $W^1$, $W^2$, $W^3$, R and Y are as defined in claim 1, which comprises reacting a compound of formula :

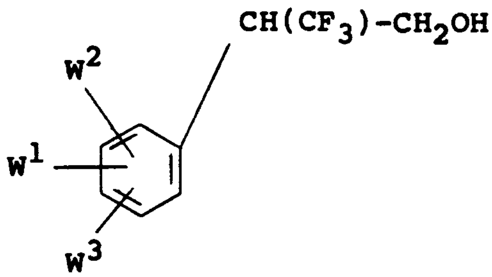

with a compound of formula :

Y-CH(R)-Q

where Q represents chlorine or bromine, or Q represents a group of formula $-OSO_2CH_3$ or a group of formula

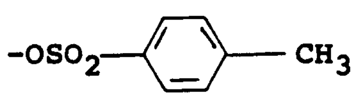

**9.** A compound of formula :

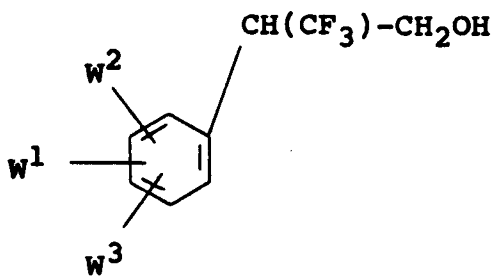

wherein $W^1$, $W^2$, and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms.

**10.** A compound of formula :

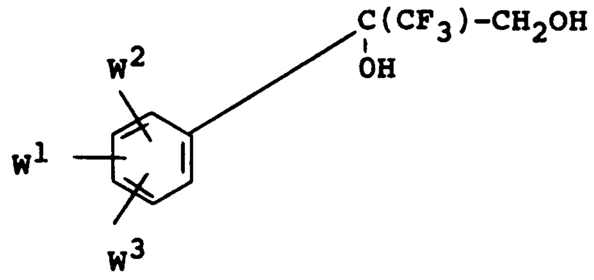

wherein $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms.

**11.** A compound of formula :

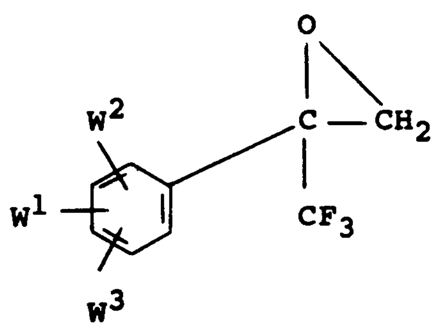

wherein $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms.

**12.** A compound of formula :

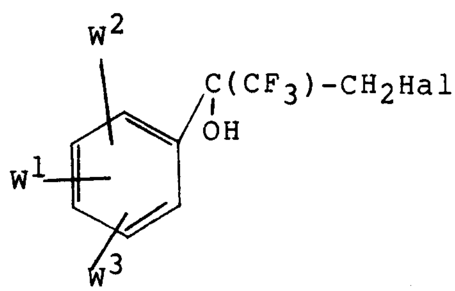

wherein Hal is chloro or bromo and $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms.

**13.** A compound of formula :

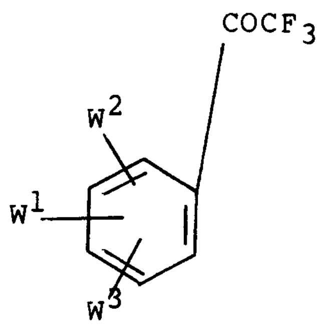

wherein $W^1$, $W^2$ and $W^3$ are independently selected from halo, alkyl of up to six carbon atoms, alkoxy of up to six carbon atoms, alkoxyalkyl of up to a total of six carbon atoms, haloalkyl of up to six carbon atoms and haloalkoxy of up to six carbon atoms; excluding 2,4,6-trimethoxy-α,α,α-trifluoroacetophenone, 2,4,6-trimethyl-α,α,α-trifluoroacetophenone and 2,4-dimethoxy-5-n-hexyl-α,α,α-trifluoroacetoacetophenone.

**14.** A compound of formula:

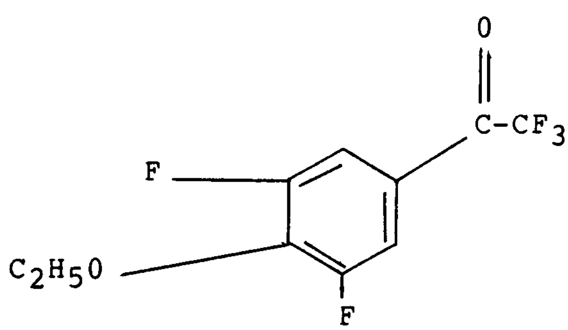

**15.** A process for preparing a product of formula:

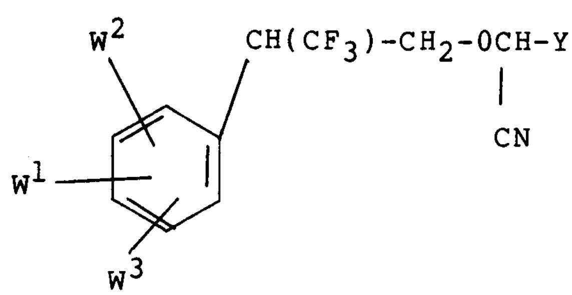

wherein $W^1$, $W^2$, $W^3$ and Y are as defined in claim 1, which comprises the steps of

(i) reacting a compound of formula

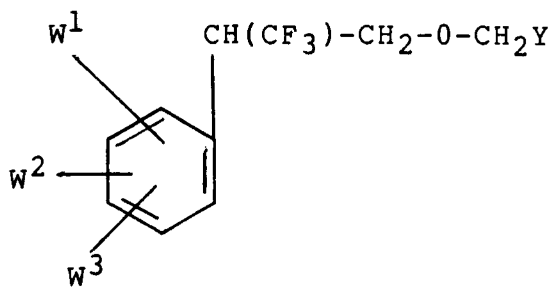

with N-bromosuccinimide in the presence of a radical initiator, followed by the steps of
(ii) reacting a compound of formula

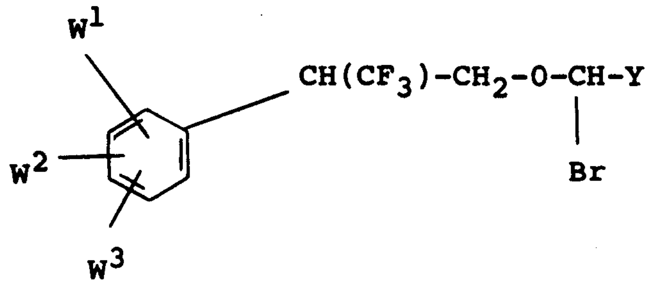

as prepared by the process of step (i), with cuprous cyanide.

**Revendications**

**1.** Composé de formule :

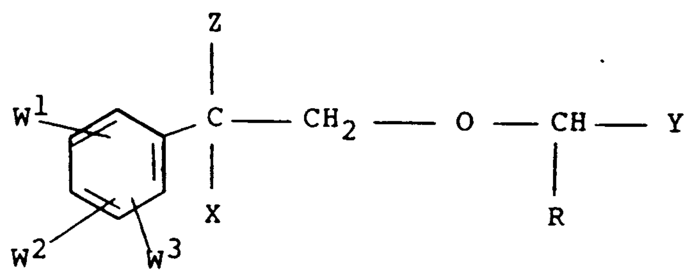

dans laquelle X est choisi entre l'hydrogène, un radical halogéno, hydroxy, alkoxy ayant jusqu'à 4 atomes de carbone et acyloxy ayant jusqu'à 4 atomes de carbone, Z représente un groupe fluoralkyle ayant un ou deux atomes de carbone, Y représente un groupe aryle substitué dont chaque substituant est choisi entre des substituants halogéno, alkyle ayant jusqu'à 6 atomes de carbone, aryle, aralkyle ayant jusqu'à 4 atomes de carbone dans la portion alkyle, aryloxy et arylamino, et

(i) R représente l'hydrogène et $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone, ou bien

(ii) R est choisi entre les radicaux méthyle, trifluorométhyle, cyano et éthynyle, et $W^1$, $W^2$ et $W^3$ sont choisis indépendamment entre l'hydrogène, des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone.

2. Composé suivant la revendication 1, dans lequel X est choisi entre l'hydrogène, un radical halogéno et un radical hydroxy, Z représente le groupe trifluorométhyle, Y représente un groupe aryle choisi entre les groupes phényle, pyridyle et furyle, substitué avec un ou plusieurs substituants choisis entre fluoro, méthyle, phényle, benzyle, phénoxy, chlorophénoxy, fluorophénoxy, bromophénoxy et fluoranilino, et
   (i) R représente l'hydrogène et $W^1$, $W^2$ et $W^3$ sont choisis indépendamment entre des radicaux fluoro, chloro, bromo, alkyle ayant jusqu'à 4 atomes de carbone, alkoxy ayant jusqu'à 4 atomes de carbone, alkoxyalkyle ayant jusqu'à un total de 4 atomes de carbone, halogénalkyle ayant jusqu'à 2 atomes de carbone et halogénalkoxy ayant jusqu'à 2 atomes de carbone, ou bien
   (ii) R est choisi entre les groupes méthyle, trifluorométhyle, cyano et éthynyle, et $W^1$, $W^2$ et $W^3$ sont choisis indépendamment entre l'hydrogène, des radicaux fluoro, chloro, bromo, alkyle ayant jusqu'à 4 atomes de carbone, alkoxy ayant jusqu'à 4 atomes de carbone, alkoxyalkyle ayant jusqu'à un total de 4 atomes de carbone, halogénalkyle ayant jusqu'à 2 atomes de carbone et halogénalkoxy ayant jusqu'à 2 atomes de carbone.

3. Composé suivant la revendication 1, dans lequel X est choisi entre l'hydrogène, les radicaux fluoro et chloro, Z représente un groupe trifluorométhyle, Y représente un groupe phénoxyphényle dont chaque groupe phényle peut être non substitué ou peut être substitué avec un halogène et R, $W^1$, $W^2$ et $W^3$ sont tels que décrits dans la revendication 1, sous réserve que l'un de W, $W^2$ et $W^3$ représente un substituant en position 4 choisi entre chloro, fluoro, méthoxy, éthoxy, trifluorométhyle, trifluorométhoxy et difluorométhoxy.

4. Composition insecticide et acaricide, comprenant une quantité à effet insecticide et acaricide d'un composé tel que défini dans la revendication 1 en association avec un diluant ou support inerte du point de vue insecticide et acaricide.

5. Procédé pour combattre des insectes et des acariens parasites en un lieu, qui consiste à appliquer au lieu une quantité à effet insecticide et acaricide d'une composition telle que définie dans la revendication 2.

6. Procédé de préparation d'un produit de formule

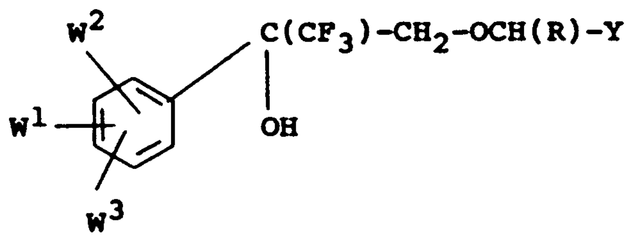

dans laquelle $W^1$, $W^2$, $W^3$, R et Y sont tels que définis dans la revendication 1, qui consiste (i) à faire réagir un composé de formule :

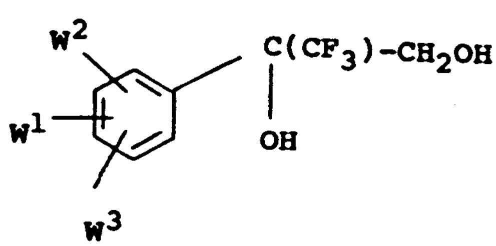

avec un composé de formule :

Y-CH(R)-Hal

ou (ii) à faire réagir un composé de formule :

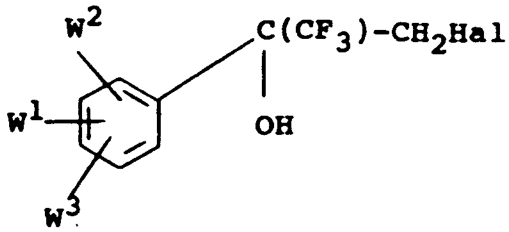

avec un composé de formule :

Y-CH(R)-OH

ou (iii) à faire réagir un composé de formule :

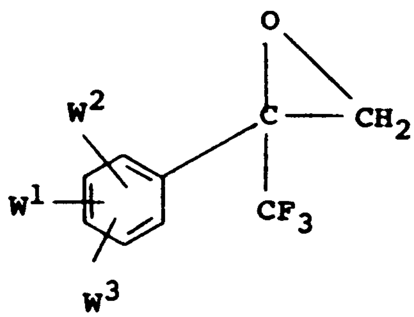

avec un composé de formule :

Y-CH(R)-OH

puis, le cas échéant, à convertir le produit

(a) en uncomposé de formule :

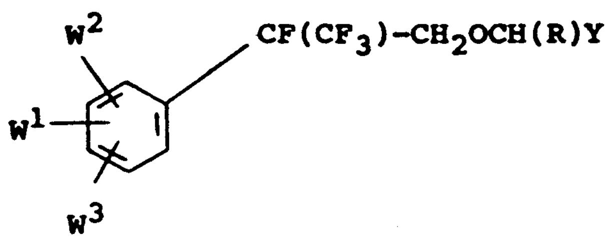

par réaction avec un agent de fluoration ; ou

(b) en un composé de formule :

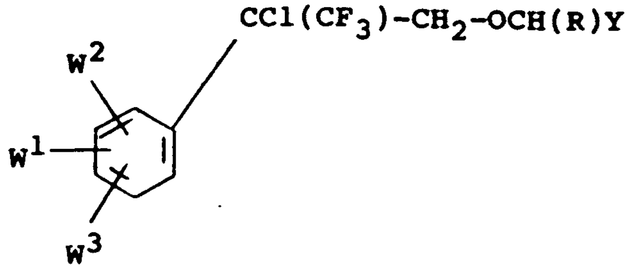

et/ou en un composé de formule :

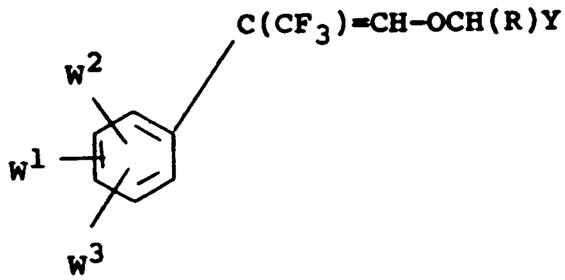

par réaction avec un chlorure d'acide inorganique, ou
(c) en un dérivé O-alkylique par réaction avec un halogénure d'alkyle et une base, ou
(d) en un dérivé O-acylique par réaction avec un chlorure d'acyle ou un anhydride d'acyle,
Hal représentant le chlore ou le brome.

**7.** Procédé de préparation d'un composé de formule

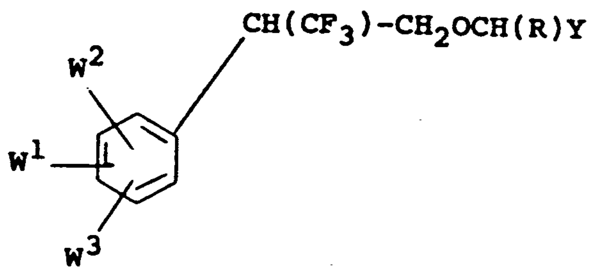

dans laquelle $W^1$, $W^2$, $W^3$, R et Y sont tels que définis dans la revendication 1, qui comprend l'élimination de chlore, par réduction, du composé correspondant de formule

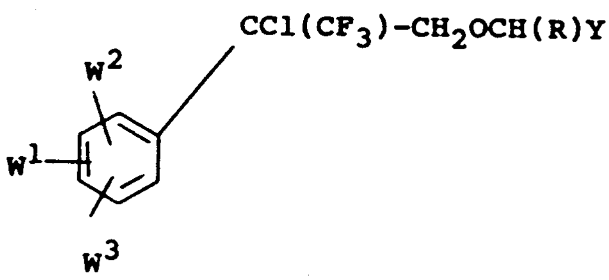

**8.** Procédé de préparation d'un composé de formule

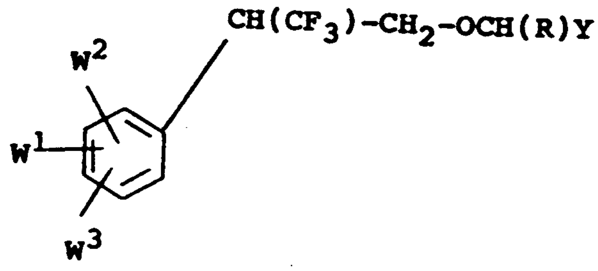

dans laquelle $W^1$, $W^2$, $W^3$, R et Y sont tels que définis dans la revendication 1, qui consiste à faire réagir un composé de formule :

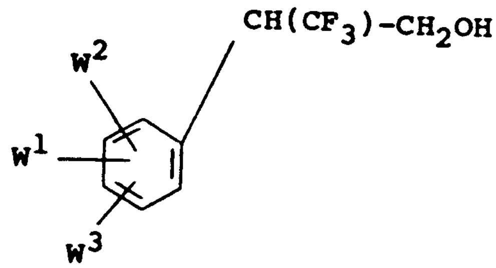

avec un composé de formule :

Y-CH(R)-Q

dans laquelle Q représente le chlore ou le brome ou bien Q représente un groupe de formule $-OSO_2CH_3$ ou un groupe de formule

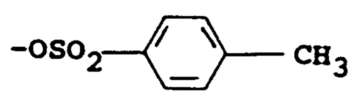

**9.** Composé de formule :

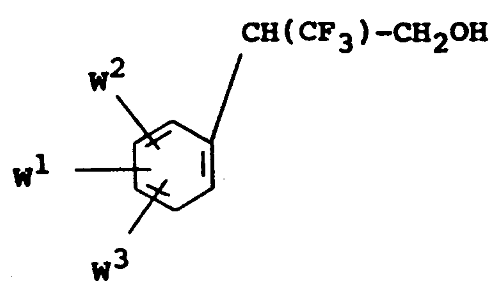

dans laquelle $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone.

**10.** Composé de formule :

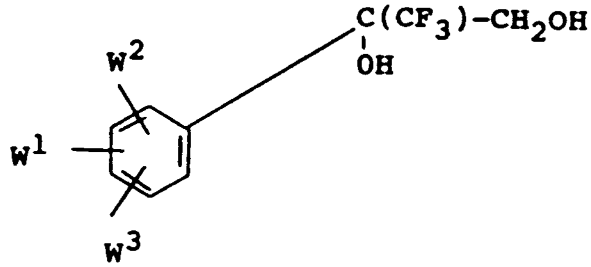

dans laquelle $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone.

**11.** Composé de formule :

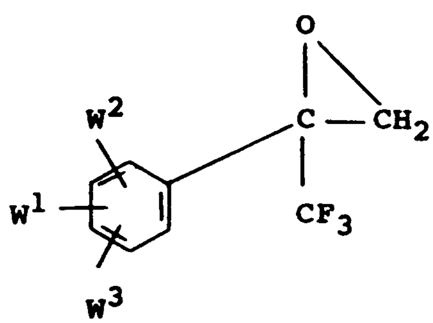

dans laquelle $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone.

**12.** Composé de formule :

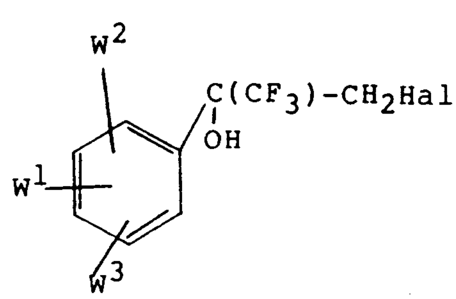

dans laquelle Hal est un radical chloro ou bromo et $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone.

**13.** Composé de formule :

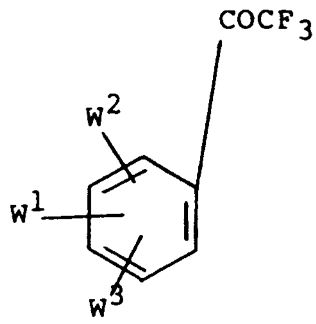

dans laquelle $W^1$, $W^2$ et $W^3$ sont choisis indépendamment entre des radicaux halogéno, alkyle ayant jusqu'à 6 atomes de carbone, alkoxy ayant jusqu'à 6 atomes de carbone, alkoxyalkyle ayant jusqu'à un total de 6 atomes de carbone, halogénalkyle ayant jusqu'à 6 atomes de carbone et halogénalkoxy ayant jusqu'à 6 atomes de carbone ; à l'exclusion de la 2,4,6-triméthoxy-α,α,α-trifluoracétophénone, de la 2,4,6-triméthyl-α,α,α-trifluoracétophénone et de la 2,4-diméthoxy-5-n-hexyl-α,α,α-trifluoracéto-acétophénone.

**14.** Composé de formule :

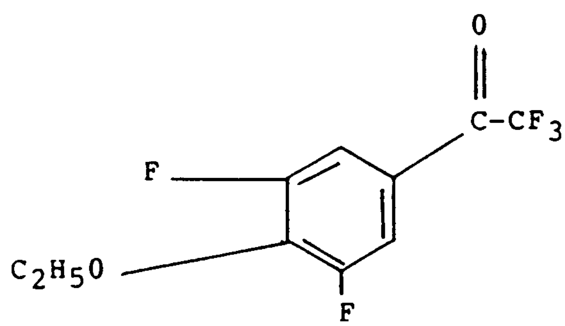

**15.** Procédé de préparation d'un produit de formule

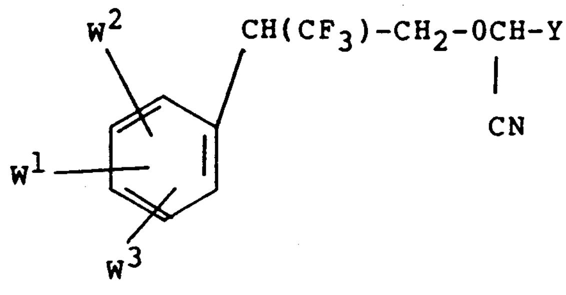

dans laquelle $W^1$, $W^2$, $W^3$ et Y sont tels que définis dans la revendication 1, qui comprend les étapes consistant :

(i) à faire réagir un composé de formule

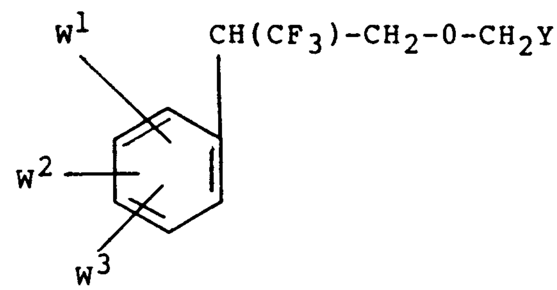

avec le N-bromosuccinimide en présence d'un initiateur radicalaire, puis
(ii) à faire réagir un composé de formule

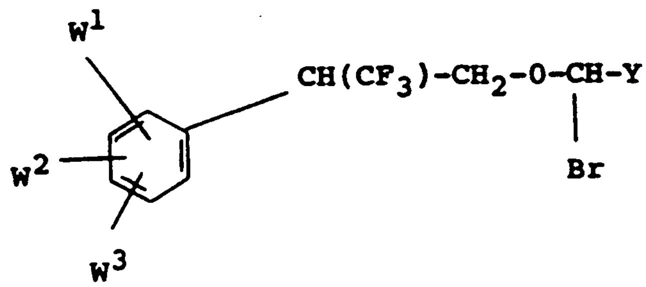

tel que préparé par le procédé de l'étape (i), avec le cyanure cuivreux.

**Patentansprüche**

1. Verbindung der Formel,

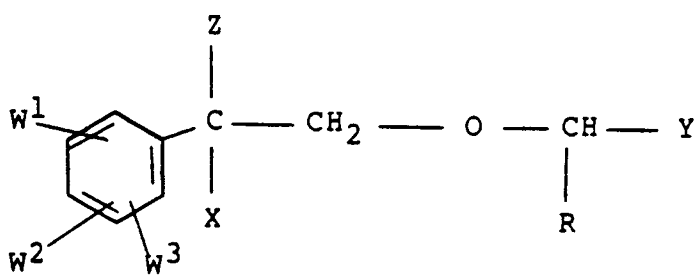

worin X ausgewählt ist aus Wasserstoff, Halogen, Hydroxy, Alkoxy mit bis zu 4 Kohlenstoffatomen und Acyloxy mit bis zu 4 Kohlenstoffatomen, Z für eine Fluoroalkyl-Gruppe mit 1 oder 2 Kohienstoffatomen steht, Y für eine substituierte Aryl-Gruppe steht, worin jeder Substituent ausgewählt ist aus Halogen, Alkyl mit bis zu 6 Kohlenstoffatomen, Aryl, Aralkyl mit bis zu 4 Kohlenstoffatomen im Alkyl-Teil, Aryloxy und Arylamino, und entweder

(i) R für Wasserstoff steht und $W^1$, $W^2$ und $W^3$ unabhängig ausgewählt sind aus Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen oder

(ii) R ausgewählt ist aus Methyl, Trifluoromethyl, Cyano und Ethinyl und $W^1$, $W^2$ und $W^3$ unabhängig ausgewählt sind aus Wasserstoff, Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen.

2. Verbindung nach Anspruch 1, worin X ausgewählt ist aus Wasserstoff, Halogen und Hydroxy, Z für die Trifluoromethyl-Gruppe steht, Y für eine Aryl-Gruppe steht, die ausgewählt ist aus Phenyl, Pyridyl und

Furyl und substituiert ist mit einem oder mehreren Substituenten, die ausgewählt sind aus Fluoro, Methyl, Phenyl, Benzyl, Phenoxy, Chlorophenoxy, Fluorophenoxy, Bromophenoxy und Fluoroanilino, und entweder

(i) R für Wasserstoff steht und $W^1$, $W^2$ und $W^3$ unabhängig ausgewählt sind aus Fluoro, Chloro, Bromo, Alkyl mit bis zu 4 Kohlenstoffatomen, Alkoxy mit bis zu 4 Kohlenstoffatomen, Alkoxyalkyl mit bis zu insgesamt 4 Kohlenstoffatomen, Halogenoalkyl mit bis zu 2 Kohlenstoffatomen und Halogenoalkoxy mit bis zu 2 Kohlenstoffatomen oder

(ii) R ausgewählt ist aus Methyl, Trifluoromethyl, Cyano und Ethinyl und $W^1$, $W^2$ und $W^3$ unabhängig ausgewählt sind aus Wasserstoff, Fluoro, Chloro, Bromo, Alkyl mit bis zu 4 Kohlenstoffatomen, Alkoxy mit bis zu 4 Kohlenstoffatomen, Alkoxyalkyl mit bis zu insgesamt 4 Kohlenstoffatomen, Halogenoalkyl mit bis zu 2 Kohlenstoffatomen und Halogenoalkoxy mit bis zu 2 Kohlenstoffatomen.

3. Verbindung nach Anspruch 1, worin X ausgewählt ist aus Wasserstoff, Fluoro und Chloro, Z für Trifluoromethyl steht, Y für eine Phenoxyphenyl-Gruppe steht, worin jede Phenyl-Gruppe unsubstituiert ist oder durch Halogen substituiert sein kann, und R, $W^1$, $W^2$ und $W^3$ die in Anspruch 1 angegebenen Bedeutungen besitzen, mit der Maßgabe, daß eines der Symbole $W^1$, $W^2$ und $W^3$ für einen Substituenten in der 4-Stellung steht, der ausgewählt ist aus Chloro, Fluoro, Methoxy, Ethoxy, Trifluoromethyl, Trifluoromethoxy und Difluoromethoxy.

4. Insecticide und acaricide Zusammensetzung, welche eine insecticid und acaricid wirksame Menge einer Verbindung nach Anspruch 1 gemeinsam mit einem insecticid und acaricid inerten Verdünnungs- oder Trägermittel enthält.

5. Verfahren zur Bekämpfung von Insekten- und Milbenschädlingen an einem bestimmten Ort, bei welchem auf den Ort eine insecticid und acaricid wirksame Menge einer Zusammensetzung nach Anspruch 2 aufgebracht wird.

6. Verfahren zur Herstellung eines Produkts der Formel,

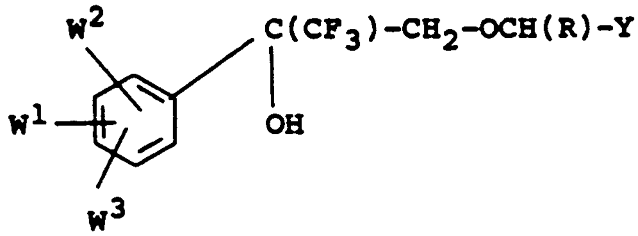

worin $W^1$, $W^2$, $W^3$, R und Y die in Anspruch 1 angegebenen Bedeutungen besitzen, bei welchem entweder (i) eine Verbindung der Formel

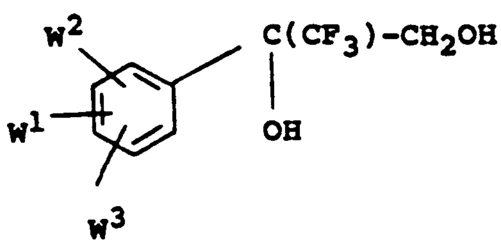

mit einer Verbindung der Formel

Y-CH(R)-Hal

oder (ii) eine Verbindung der Formel

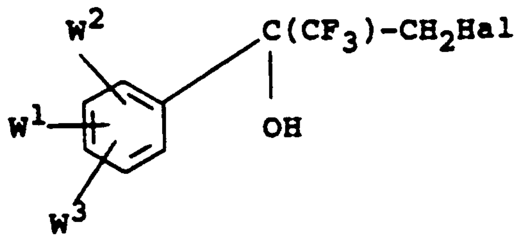

mit einer Verbindung der Formel

Y-CH(R)-OH

oder (iii) eine Verbindung der Formel

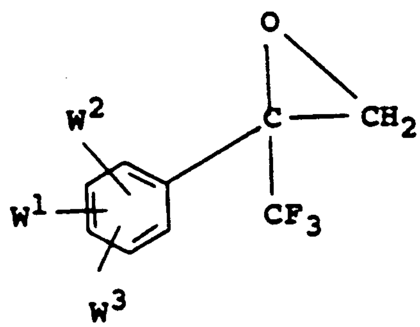

mit einer Verbindung der Formel

Y-CH(R)-OH

umgesetzt wird und hierauf gegebenenfalls das Produkt
(a) durch Umsetzung mit einem Fluorierungsmittel in eine Verbindung der Formel

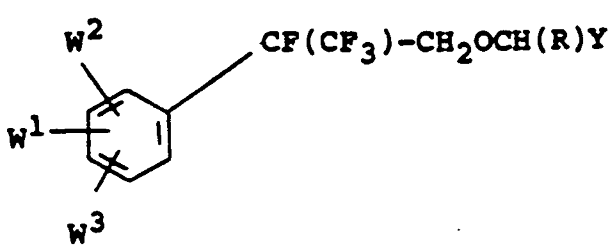

oder

(b) durch Umsetzung mit einem anorganischen Säurechlorid in eine Verbindung der Formel

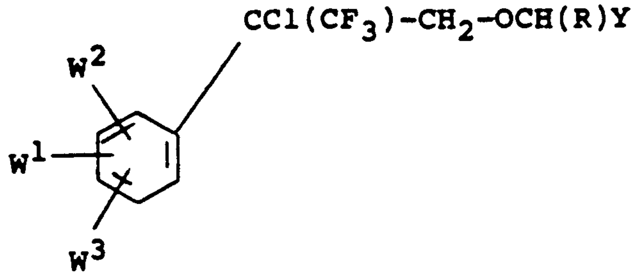

und/oder eine Verbindung der Formel

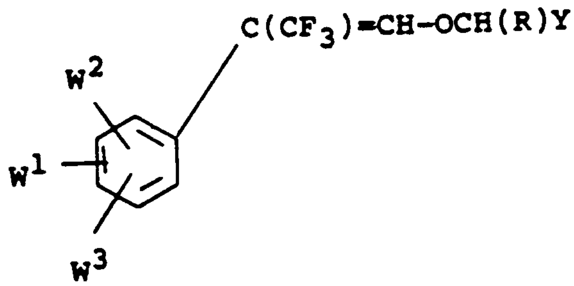

oder

(c) durch Umsetzung mit einem Alkylhalogenid und einer Base in ein O-Alkyl-Derivat oder

(d) oder durch Umsetzung mit einem Acylchlorid oder einem Acylanhydrid in ein O-Acyl-Derivat umgewandelt wird, wobei Hal für Chlor oder Brom steht.

**7.** Verfahren zur Herstellung einer Verbindung der Formel,

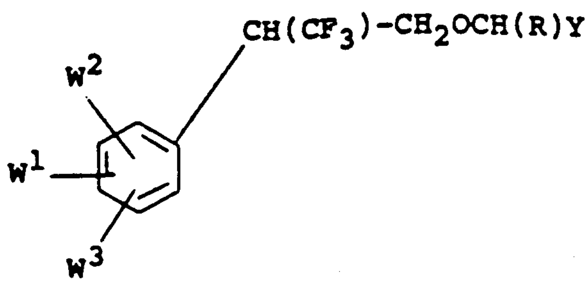

worin $W^1$, $W^2$, $W^3$, R und Y die in Anspruch 1 angegebenen Bedeutungen besitzen, bei welchem die entsprechende Verbindung der Formel

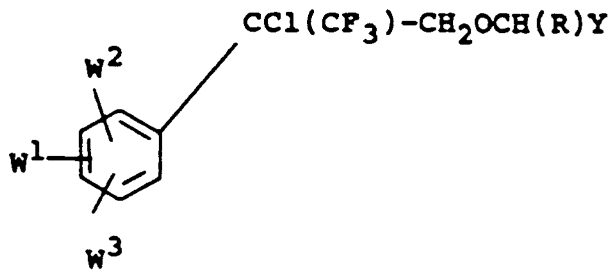

einer reduktiven Chlorierung unterworfen wird.

**8.** Verfahren zur Herstellung einer Verbindung der Formel,

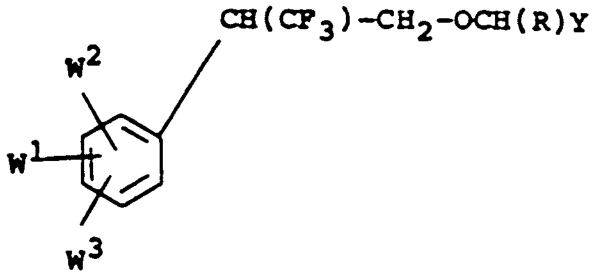

worin $W^1$, $W^2$, $W^3$, R und Y die in Anspruch 1 angegebenen Bedeutungen besitzen, bei welchem eine Verbindung der Formel

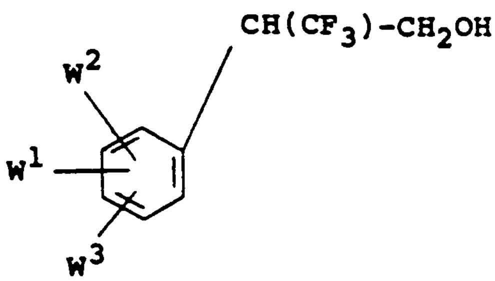

mit einer Verbindung

Y-CH(R)-Q

umgesetzt wird,
wobei Q für Chlor oder Brom steht oder Q für eine Gruppe der Formel $-OSO_2CH_3$ oder eine Gruppe der Formel

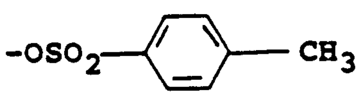

steht.

**9.** Verbindung der Formel,

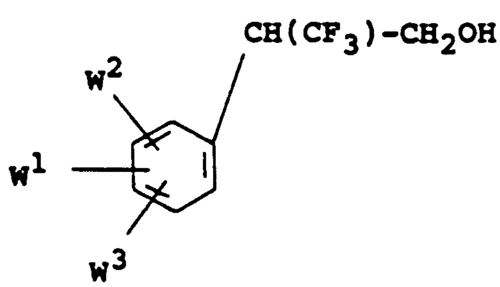

worin $W^1$, $W^2$ und $W^3$ unabhängig ausgewählt sind aus Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen.

**10.** Verbindung der Formel,

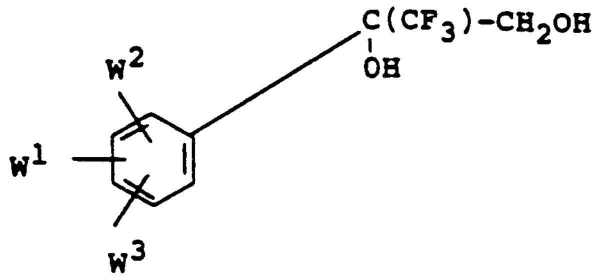

worin $W^1$, $W^2$ und $W^3$ unabhängig ausgewählt sind aus Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen.

**11.** Verbindung der Formel,

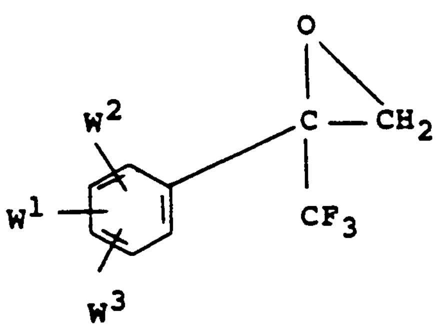

worin $W^1$, $W^2$ und $W^3$ unabhängig ausgewählt sind aus Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen.

**12.** Verbindung der Formel,

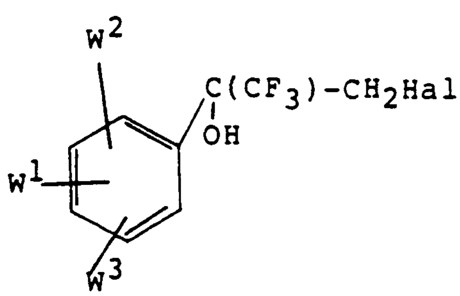

worin Hal für Chloro oder Bromo steht und $W^1$, $W^2$ und $W^3$ unabhängig ausgewählt sind aus Halogen, Alkyl mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen.

**13.** Verbindung der Formel,

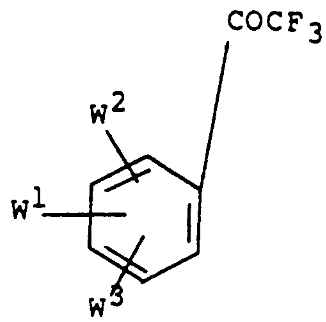

worin $W^1$, $W^2$ und $W^3$ unabhängig ausgewählt sind aus Halogen, Alkyl mit bis zu 6 Kohlenstoffatomen, Alkoxy mit bis zu 6 Kohlenstoffatomen, Alkoxyalkyl mit bis zu insgesamt 6 Kohlenstoffatomen, Halogenoalkyl mit bis zu 6 Kohlenstoffatomen und Halogenoalkoxy mit bis zu 6 Kohlenstoffatomen, wobei jedoch 2,4,6-Trimethoxy-α,α,α-trifluoroacetophenon, 2,4,6-Trimethyl-α,α,α-trifluoroacetophenon und 2,4-Dimethoxy-5-n-hexyl-α,α,α-trifluoroacetophenon ausgenommen sind.

**14.** Verbindung der Formel

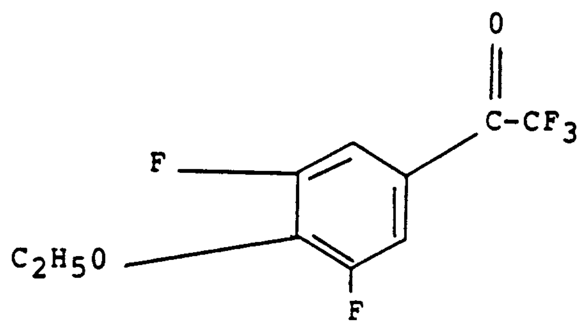

**15.** Verfahren zur Herstellung eines Produkts der Formel,

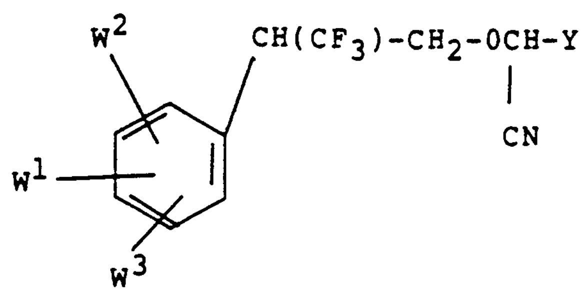

worin $W^1$, $W^2$, $W^3$ und Y die in Anspruch 1 angegebenen Bedeutungen besitzen, bei welchem
(i) eine Verbindung der Formel

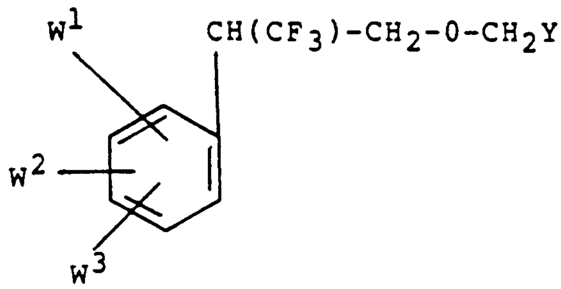

in Gegenwart eines radikalischen Initiators mit N-Bromosuccinimid umgesetzt wird und hierauf (ii) eine gemäß dem Verfahren der Stufe (i) hergestellte Verbindung der Formel

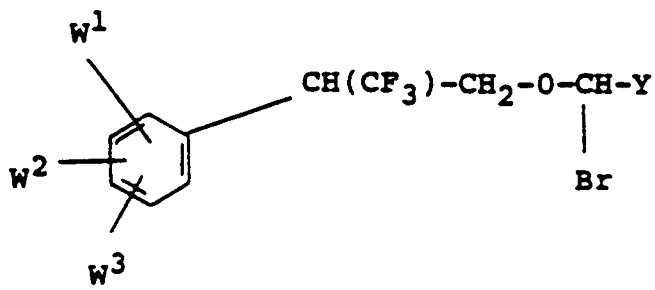

mit Kupfer(I)-cyanid umgesetzt wird.